# EUROPEAN PATENT SPECIFICATION

(11) **EP 0 528 870 B1**
(45) Date of publication and mention of the grant of the patent: **02.12.1998**
(21) Application number: 91908991.2
(22) Date of filing: 06.05.1991
(51) Int. Cl.: G01N 33/539, C12Q 1/68

(54) **PROTEIN-NUCLEIC ACID PROBES AND IMMUNOASSAYS USING SAME**
PROTEIN-NUKLEINSÄURESONDEN UND SELBIGE VERWENDENDE IMMUNOASSAYS
SONDES DE PROTEINE-ACIDE NUCLEIQUE ET DOSAGES IMMUNOLOGIQUES LES UTILISANT

(30) Priority: 04.05.1990 US 519212
(43) Date of publication of application: 03.03.1993
(73) Proprietor: CHIRON CORPORATION, Emeryville, California 94608 (US)
(72) Inventor: URDEA, Michael, S., Alamo, CA 94507 (US)
(74) Representative: Goldin, Douglas Michael (GB)
(86) International application number: US9102925
(87) International publication number: WO9117442

(56) References cited:
- EP-A- 0 154 884
- EP-A- 0 225 807
- EP-A- 0 317 077
- WO-A-87/06270
- WO-A-90/02819
- GB-A- 2 125 964
- US-A- 4 299 916
- US-A- 4 563 419
- US-A- 4 749 647
- US-A- 4 828 979
- US-A- 4 882 269
- US-A- 4 910 300
- Nucleic Acids Research, Volume 14, Number 14, issued 1986, CHU et al., "Synthesis of an amplifieable reporter RNA for bioassays", pages 5591-5603, see especially the Abstract.
- Nucleic Acids Research, Volume 14, Number 2, issued 1986, SYVANEN et al., "Fast quantitation of nucleic acid hybrids by affinite-based hybrid collection", pages 5037-5048, see especially the Introduction on pages 5037-5038.
- Nucleic Acids Research, Volume 16, Number 23, issued 1988, SYVANEN et al., "Quantitation of polymerase chain reaction products by affinity-based hybrid collection", pages 11327-11338, see especially the Abstract.

## Description

### Technical Field

This invention relates generally to the fields of immunology and nucleic acid chemistry. More specifically, it relates to the use of nucleic acid /polypeptide hybrid probes as a means of amplifying the detectable signal in immunoassays.

### Background Art

Nucleic acid hybridizations are now commonly used in genetic research, biomedical research and clinical diagnostics to detect and quantify particular nucleotide sequences which are present in heterogenous mixtures of DNA, RNA, and/or other materials. In the basic nucleic acid hybridization assay, single-stranded analyte nucleic acid (either DNA or RNA) is hybridized, directly or indirectly, to a labeled nucleic acid probe, and the duplexes containing label are quantified. Both radioactive and non-radioactive labels have been used.

The basic assay lacks sensitivity. When the analyte is present in low copy number or dilute concentration the signal cannot be distinguished from the background noise. Variations of the basic scheme have been developed to facilitate separation of the target duplexes from extraneous material and/or to amplify the analyte sequences in order to facilitate detection, but these variations have suffered generally from complex and time consuming procedures, high background, low sensitivity, and difficulty in quantification. For a general discussion of these variations see International Patent Pub. No. 89/03891.

Immunoassays are also commonly used in genetic research, biomedical research and clinical diagnostics to detect and quantify particular antigenic epitopes which are present in heterogenous mixtures of blood samples, cellular extracts and other materials. A basic immunoassay involves the specific binding of an antibody, either monoclonal or polyclonal, to a target antigen and a means for detecting the reaction. Such means have been incorporated into various assays including direct, indirect and sandwich immunoassays. However, the sensitivity of detection of the essential antigen/antibody interaction has presented much the same problems as described above for nucleic acid hybridizations.

Various techniques such as radioimmunoassay, immunoperoxidase, and ELISA are presently used for immunoassays. However, radioimmunoassays have the disadvantage of requiring the use of dangerous and environmentally unsound reagents and immunoperoxidase and ELISA suffer from a low signal to noise ratio and are limited in signal amplification.

The present invention provides signal amplification methods for immunoassays utilizing polynucleotide molecules, which have proved useful in nucleic acid hybridization assays, as part of a hybrid protein/nucleic acid probe. These amplifier probes provide a highly reproducible gain in signal, a highly reproducible signal-to-noise ratio, are themselves quantifiable and reproducible, and are capable of combining specifically with an analyte antigen present at low concentration. In one embodiment, the amplifier probes also hybridze with "universal" nucleic acid reporter moieties to form stable complexes.

Commonly owned US Patent No. 4,868,105, issued 19 September 1989, describes a solution phase hybridization sandwich assay in which the analyte nucleic acid is hybridized to a "labeling probe" and to a "capturing probe". The probe-analyte complex is coupled by hybridization to a solid-support. This permits the analyte oligonucleotide to be removed from solution as a solid phase complex, thereby concentrating the analyte, facilitating its separation from other reagents, and enhancing its subsequent detection.

Commonly owned PCT Application W090/13667 published 15 November 1990 claims linear and branched multimeric polynucleotide probes which have two domains, and methods of use of these probes as signal amplifiers in nucleic acid hybridization assays. The first domain is complementary to a single-stranded oligonucleotide sequence of interest, and the second domain is comprised of a multiplicity of single-stranded oligonucleotide subunits that are complementary to a single-stranded labeled oligonucleotide.

Commonly owned PCT Application, PCT/US91/00213 which has an earlier priority date but was not published until 25th July 1991 as W091/10746, discloses polynucleotide probes which have three domains, and methods of use of these probes as signal amplifiers in nucleic acid hybridization assays. The first domain is complementary to a single-stranded oligonucleotide sequence of interest, the second domain is capable of functioning as a promoter for a bacterial phage DNA-dependent RNA polymerase enzyme activity, and the third domain is capable of functioning as a transcriptional template for the polymerase activity.

Published PCT Application W090/02819 describes methods for detecting a target nucleic acid employing a probe comprising a first domain, which is an oligonucleotide sequence capable of hybridizing to the target, and a second domain, which can be cleaved off and is a moiety capable of initiating a transcription process, i.e. either a DNA-dependent RNA polymerase or an oligonucleotide comprising a promoter strand. Methods are also described in the same application for detecting an antigen on a solid support using an antibody linked by disulphide bonds to either a DNA-dependent RNA polymerase or the minus strand of a promoter. In the latter case, detection of the analyte involves cleavage of the hybrid probe to release the promoter strand and hybridization of that promoter strand to a second oligonucleotide comprising the positive strand of the promoter joined to a coding sequence for a replicatable RNA.

Protein/DNA hybrid molecules have been used as probes in nucleic acid hybridization assays where the protein moiety functions as a labeling component (Czichos, J., et al, Nucl. Acids Res. (1989) 17:1563; U.S. Patent No. 4,873,187; U.S. Patent No. 4,737,454).

Other forms of protein/DNA hybrid molecules have also been used as probes in immunoassays. U.S. Patent No. 4,692,509 describes a radioactively labeled hybrid probe comprising a protein and a covalently linked radioactive oligonucleotide. The radioactive moiety is used to indicate the presence of the protein moiety in a biological assay. EP-A no. 154884 discloses a protein/DNA hybrid molecule in which the protein moiety specifically recognizes a target protein and the nucleic acid moiety provides a labeling function. EP-A no. 317077, and corresponding published PCT application W089/03891 already mentioned above, disclose a solid phase immunoassay employing a protein/DNA hybrid probe comprising an antibody covalently attached to a single-stranded oligonucleotide, which is designed to hybridize with an oligonucleotide multimer.

### Summary of the Invention

One aspect of the invention is a polypeptide/polynucleotide-hybrid molecular probe for use as an amplifier of the detectable signal in immunoassays. In one embodiment the "polymerase probe" comprises three domains:
(a) a first domain (A) which is a polypeptide and functions as an antibody specific for a known antigen;
(b) a second domain (B) which is a double-stranded polydeoxyribonucleotide capable of functioning as a promoter for a DNA-dependent RNA polymerase enzyme activity; and
(c) a third domain (C) which is either single- or double-stranded and adjacent to the second domain, such that the third domain is capable of functioning as a template for the promoter activity of the second domain.

A second embodiment is a polypeptide/polynucleotide-hybrid molecular probe for use as an amplifier of the detectable signal in immunoassays. This "multimer probe" comprises two domains:
(a) a first domain (A) which is a polypeptide capable of functioning as an antibody which binds specifically to a known antigen;
(b) a second domain (M) comprising a multiplicity of single-stranded oligonucleotide subunits that are capable of binding specifically to a single-stranded nucleic acid sequence of interest, said multiplicity of single-stranded oligonucleotide subunits being covalently linked to each other; and
(c) a means for conjugating the first and second domains.

Another aspect of the invention is a method of amplifying a detectable signal in an immunoassay by use of the polymerase probes described supra. This method comprises:
(a) immobilizing the antigenic analyte, directly or indirectly, on a solid substrate;
(b) directly or indirectly binding to the analyte a first molecular probe comprising:
   (i) a binding domain (A) which is a polypeptide;
   (ii) a second domain (B) which is a double stranded DNA sequence capable of functioning as a promoter for a DNA-dependent RNA polymerase enzyme activity; and
   (iii) a third domain (C) which is either single- or double-stranded and adjacent to domain B, such that the third domain is capable of functioning as a template for the promoter activity of the second domain;
(c) removing the unbound probe;
(d) transcribing multiple copies of RNA oligomers which are complementary to the template sequence, c', of the third domain, C, of the probe construct via a DNA-dependent RNA polymerase activity; and
(e) quantifying the transcripts.

Another aspect of the invention is another method of amplifying a detectable signal in an immunoassay by use of the multimer probes described supra. This method comprises:
(a) immobilizing the analyte, directly or indirectly on a solid substrate;
(b) directly or indirectly binding to the analyte a hybrid molecular probe comprising:
   (i) a binding domain (A) which is a polypeptide, for example capable of functioning as an antibody, covalently attached to
   (ii) a second domain (M) comprising a multiplicity of single-stranded DNA oligonucleotide subunits that are capable of binding specifically to a single-stranded labeled oligonucleotide, said multiplicity of single-stranded oligonucleotide subunits being covalently linked to each other; and
   (iii) a means for conjugating the first and second domains;
(c) removing unbound probe;
(d) hybridizing single-stranded labeled oligonucleotides which comprise a nucleotide sequence which is substantially complimentary to the subunit sequence of the domain M probe to the subunits of the second domain;
(e) removing unbound labeled oligonucleotide; and
(f) quantifying the amount of labeled oligonucleotide bound to the probe.

Yet another embodiment is a method of amplifying a detectable signal in a competitive immunoassay which comprises:
(a) providing a hybrid molecule in which domain A is a polypeptide capable of functioning as a hapten, and this domain is conjugated either to a B/C domain to provide a polymerase probe or an M domain to provide a multimer probe;
(b) immobilizing the hybrid molecule, directly or indirectly upon a solid substrate in the presence of a competing analyte;
(c) removing the unbound probe; and either
(d) transcribing multiple copies of RNA oligomers via a DNA-dependent RNA polymerase activity and quantifying the transcripts - this in the instance that a polymerase probe is utilized; or
(e) hybridizing single-stranded labeled oligonucleotides, to the subunits of the M domain, removing the unbound label and quantifying the bound label - this in the instance that a multimer probe is utilized.

### Brief Description of the Drawings

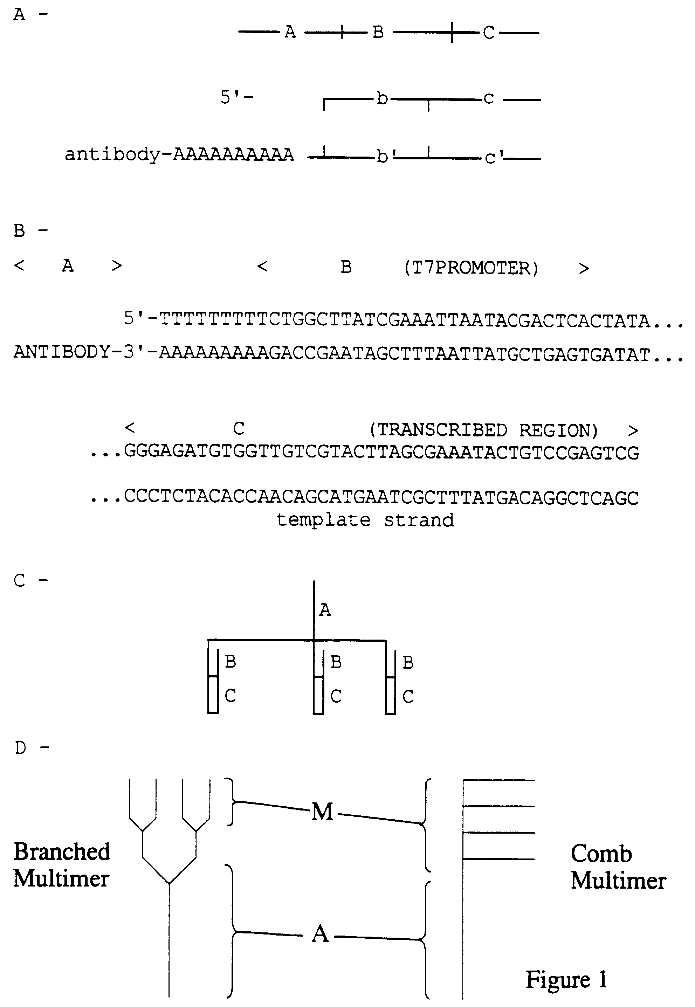

Figure 1A is a schematic representation of a monomeric polymerase-type amplifier probe. Capital letters designate domains, and lower case letters designate strands within a domain. A primed letter designates a lower strand (read 3'- to 5'-, left to right). The A domain is an antibody which recognizes a target epitope. The B domain is the promoter for a RNA polymerase. The c' sequence is the template for the RNA polymerase. The probe is synthesized as a single strand. The AAAAAAA at the end of the C region represents the poly-A linker added to allow for self-annealing. The A/T region between the A and B domain is optionally required to retain full antibody activity.

Figure 1B is the DNA sequence of one embodiment of the polymerase-type amplifier probe. The promoter domain, B, consists of the consensus sequence of the bacteriophage T7 promoter (5'-TAATACGACTCACTATA-3') plus 15 additional residues 5' to the promoter sequence.

Figure 1C is a schematic representation of a multimeric polymerase-type amplifier probe in which the A domain functions as an antibody and the double stranded B and C domains are self- annealing.

Figure 1D is a schematic representation of multimeric amplifier probes in which the A domain functions as an antibody and the M domain is comprised of multiple oligomeric subunits. Both branched and comb-like multimers are depicted.

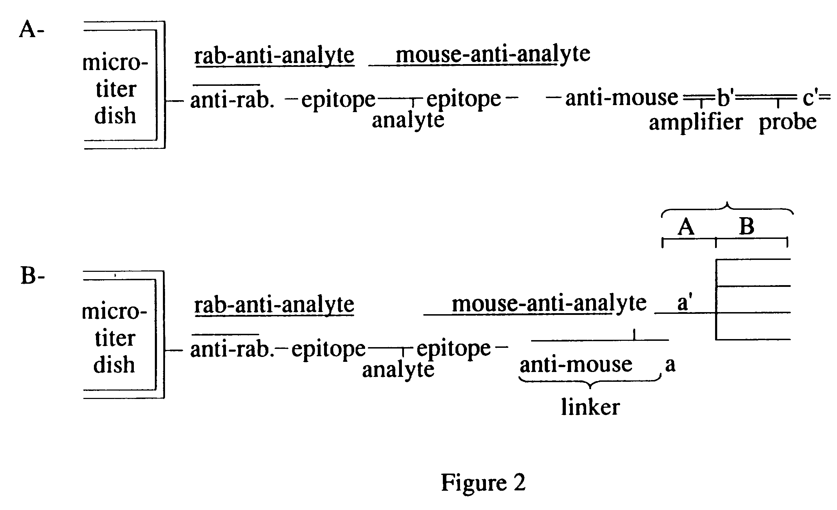

Figure 2A is a schematic representation of a sandwich immunoassay system which incorporates the polymerase-type amplifier probe. The analyte protein is indirectly immobilized upon a solid substrate by complexing with a first antibody (e.g. a "rab" (rabbit) - antibody); and indirectly joined to the amplifier probe by complexing with a second antibody (e.g. a mouse antibody).

Figure 2B is a schematic representation of a sandwich hybridization/immunoassay system. The analyte protein is indirectly immobilized as above. The second antibody (mouse-anti-analyte) is complexed with a hybrid antibody/polynucleotide molecule in which the protein moiety functions as an anti-mouse antibody and the polynucleotide moiety is complementary to the a' nucleotide region of the amplifier probe (in this case, a multimer probe).

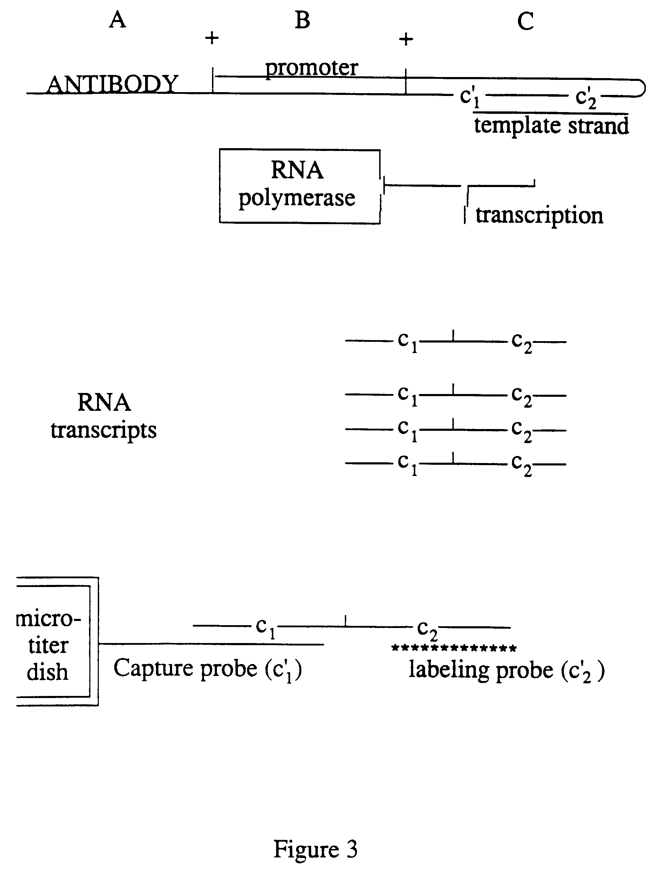

Figure 3 depicts the use of RNA polymerase transcripts as reporter molecules in an immunoassay. After the sandwich complex which incorporates the polymerase probe is formed, RNA polymerase is added and multiple RNA transcripts (c) complementary to the template sequence (c') are produced. These sequences have two sub-domains: c1 which is complementary to a capture probe, immobilized upon a solid substrate; and c2 which is complementary to a labelling probe. This allows for indirect immobilization of the label and easy quantification of the hybridization assay signal. "*" designates the incorporated label which may be radioactive, chemiluminescent, fluorescent or enzymatic.

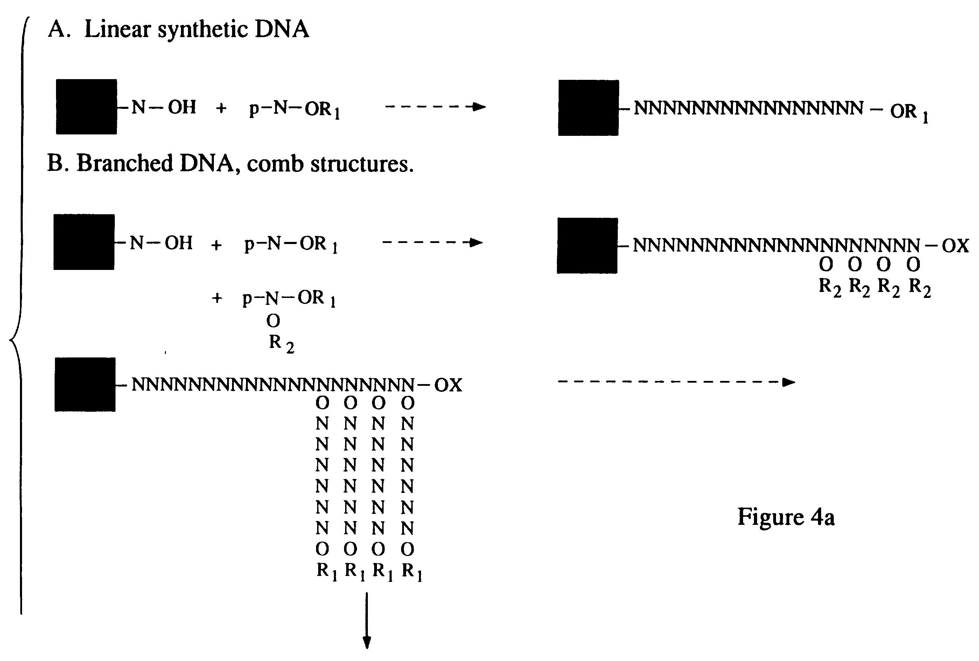

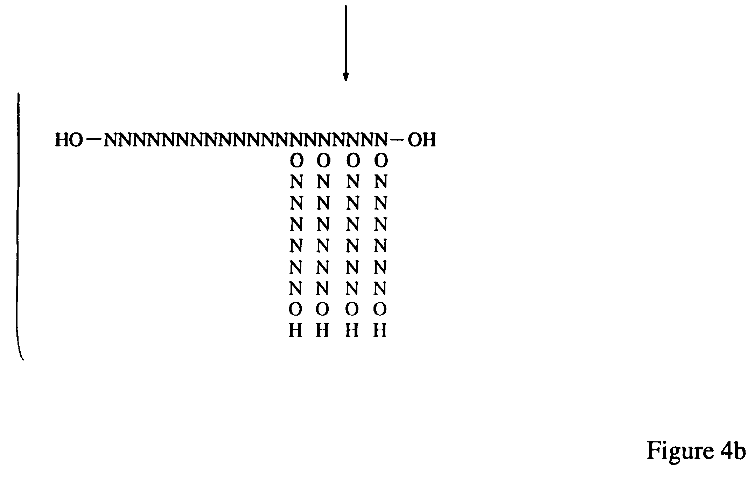

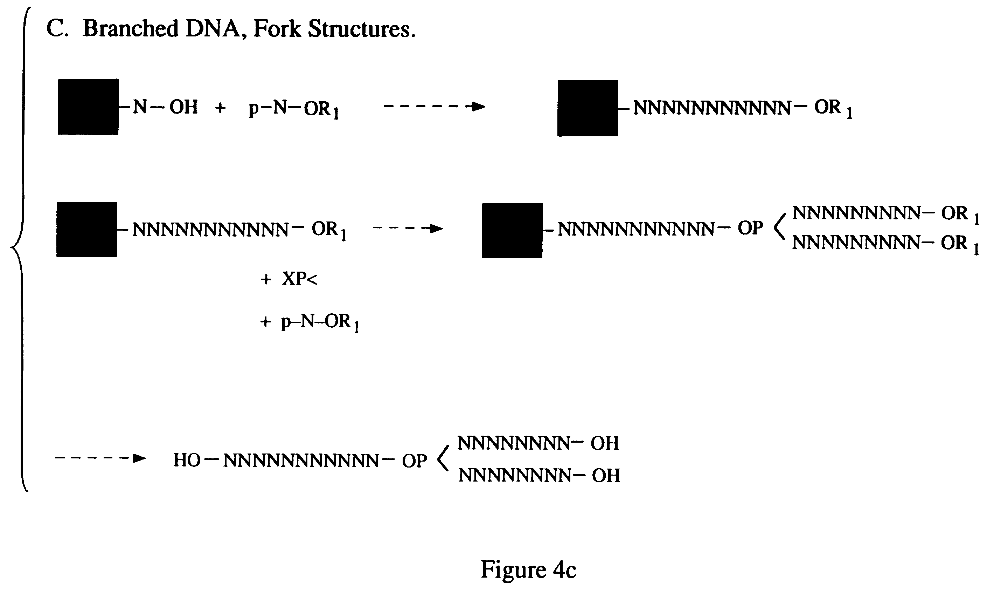

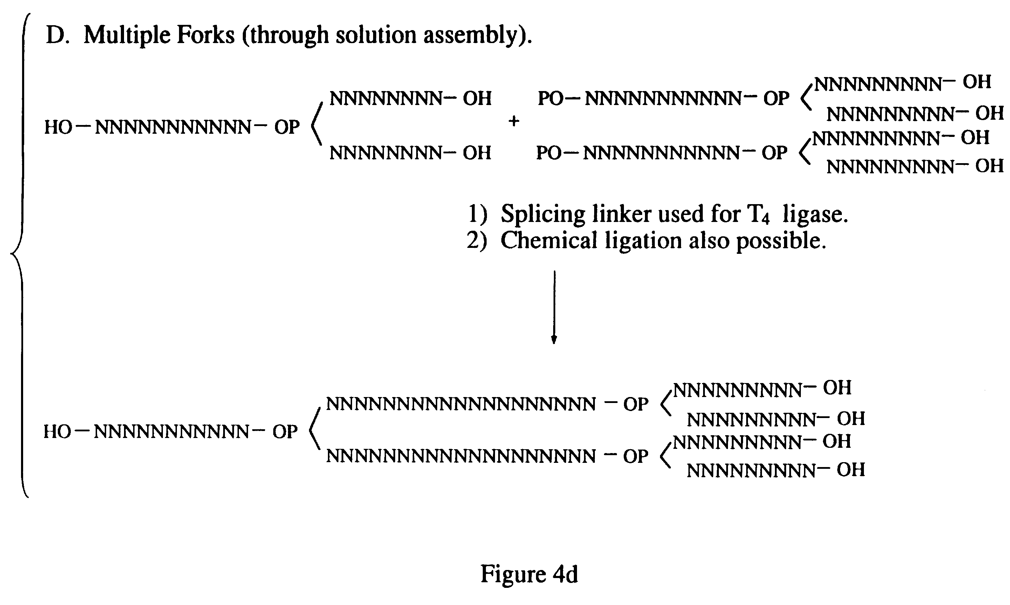

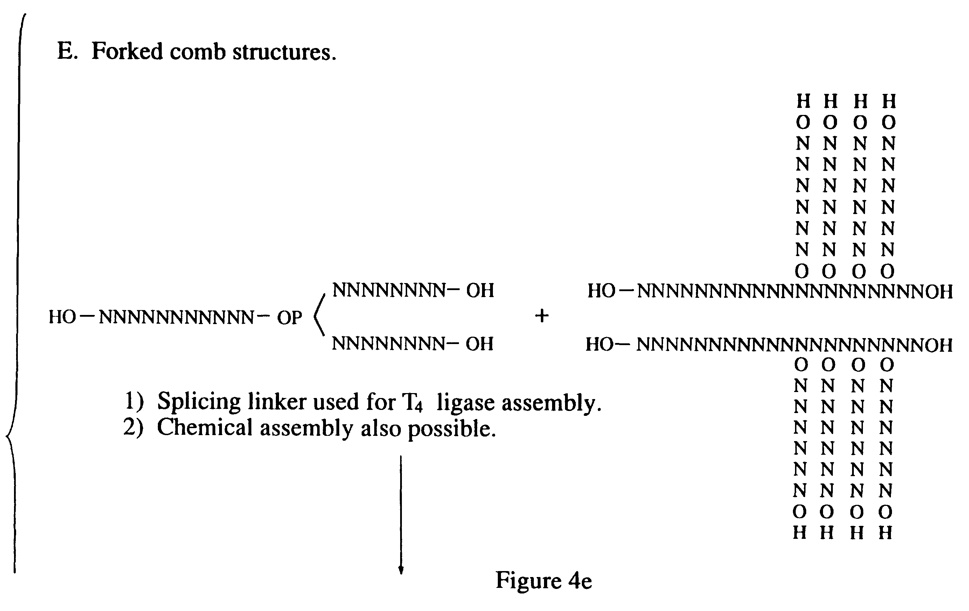

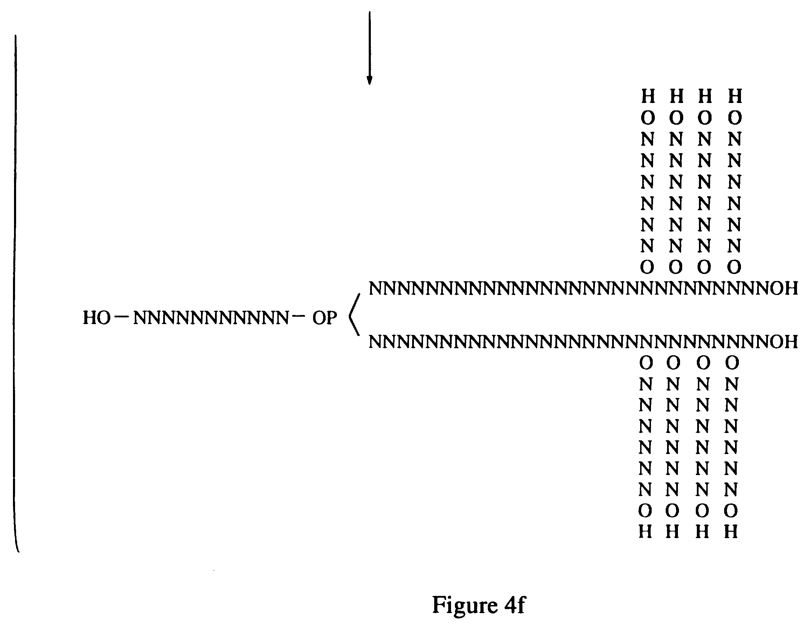

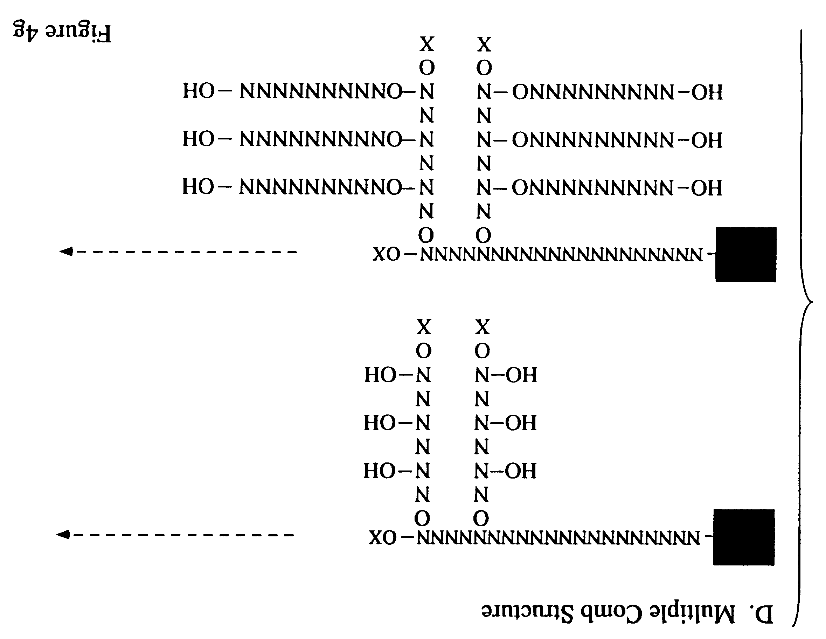

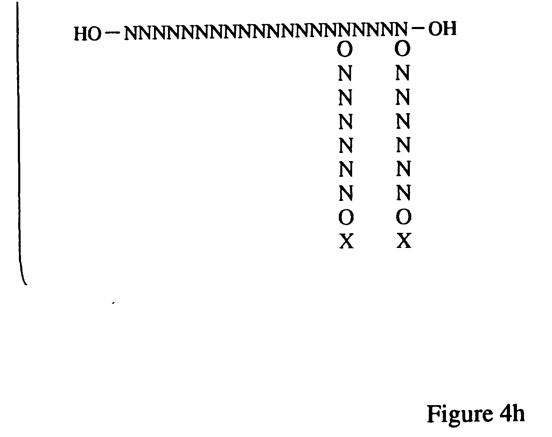

Figure 4 (Parts A-F) illustrate procedures used in making multimers having "comb-like" and/or bifurcated structures.

### Detailed Description of the Invention

### Definitions

A "**detectable signal**" is a transmissible indicium of the occurrence of a biochemical event such as a nucleic acid hybridization or the binding of an antigen and antibody. The subject application describes methods of amplifying the detectable signal of immunoassays.

**"DNA-dependent RNA polymerase"** is an enzyme which facilitates the polymerization of RNA of specific sequence from a complementary DNA template.

A "**domain**" is a particular region of a biochemical molecule characterized by its function.

An "**epitope"** is that portion of an immunogenic molecule that is specifically recognized by, and complexes with, its corresponding antibody in an immunological reaction.

A nucleotide/peptide "**hybrid**" molecule comprise both nucleic acid residues and amino acid residues, each in a separate functional domain of the molecule.

An "**immunogen**" is a substance which can react in a specific fashion with an antibody.

An **"immunological reaction"** is the specific recognition and binding of an antibody to the epitope of an immunogen. An "**immunoassay**" is a method of determining the presence of an epitope by combining an immunological reaction with a means for detecting and quantifying the reaction.

A **"polydeoxyribonucleotide"** is a polymeric DNA molecule. A **"polynucleotide"** is a polymeric DNA or RNA molecule.

A "**promoter**" is the site on a polydeoxyribonucleotide to which a RNA polymerase enzyme binds preparatory to initiating transcription.

"**RNA-dependent RNA polymerase"** is an enzyme which facilitates the polymerization of RNA of specific sequence from a complementary RNA template.

"**Transcription**" is an process, mediated by an enzyme, by which RNA is formed from a complementary polynucleotide template.

The "**upper strand**" of a double-stranded DNA molecule is the strand whose 5'-end is on the left as the sequence is read from left to right. The sequence of this strand is always presented above the sequence for its complementary "**lower strand**" which is read 3'- to 5'-, left to right.

### Modes for Carrying Out the Invention

### I - Amplification Probes

### A- Polymerase Probes

One aspect of this invention is a DNA amplification probe (referred to as a "polymerase probe") containing three functional domains. This probe is used to enhance the detectable signal in immunoassays.

In a preferred embodiment, the first domain (A - Figure 1A) is a polypeptide which functions as an antibody with specificity for an antigen of choice. The functional region of the polypeptide is followed by a region of either amino acid or nucleic acid residues, i.e. - a spacer region, which does not substantially interfere with the activity of the antibody. The antibody activity can be specific for an epitope of the analyte itself. However, in a preferred embodiment, the antibody is directed to an antigenic determinant of the particular immunoglobin used in the assay described infra (e.g. anti-rabbit IgG, or anti-human IgG - See Figure 2A). As applied, the analyte is contacted with a specific antibody, excess antibody is removed and the amplification probe is then allowed to react with the bound antibody.

Preferably, the analyte is first immobilized upon a solid substrate to facilitate subsequent washing procedures. This immobilization may be direct (e.g. - biological preparations containing the analyte might be bound to a nitrocellulose filter) or indirect (e.g. - a specific antibody might be immobilized on a solid substrate and the analyte subsequently bound to the immobilized antibody).

The second domain (B - Figure 1A), usually 10 to 40 base pairs in length, preferably 20 to 35 base pairs, more preferably 30 to 35 base pairs, is double stranded and functions as a DNA-directed RNA polymerase promoter. This promoter is usually derived from the promoter sequence of a bacterial phage, preferably any of the phage T3, T7, or SP6, more preferably from bacteriophage T7. This class of RNA polymerases is highly promoter specific. The T7 promoter is probably the best characterized (Figure 1B). DNA sequences from 17 T7 promoters are known and a consensus sequence has been deduced: 5'-TAATACGACTCACTATA-3' (Oakley and Coleman, Proc. Nat. Acad. Sci. (1977) 74:4266; Dunn and Studier, J. Molec. Biol. (1983) 166:477). Sequences 3' to the promoter on the complementary strand (the c' segment, whose 3' end is adjacent to the 5' end of the b' segment) serve as the template for transcription and the transcription of many template sequence variations can be accommodated (Figure 1B). Only the promoter region itself must be double-stranded (Milligan et al, Nuc. Acids Res. (1987) 15:8783).

Extensions at the 5' end of the promoter have little effect on transcription. For example, in a preferred embodiment, the B region consists of the consensus sequence of the T7 promoter plus additional bases 5' to the consensus sequence which are identical to the sequence of the pT7 plasmids (available from US Biochemicals) up to the Pvu II restriction site (Figure 1B). These sequences may or may not be extraneous.

The third domain (C - Figure 1A) is directly 3' to the second domain and the c' strand of this domain serves as the template for the domain B promoter. Domain C is usually 30 to 80 nucleotides in length, preferably 35 to 50 nucleotides, most preferably 40 to 45 nucleotides. This domain may be either single or double stranded The 3' end of the c' template strand (directly adjacent to the promoter) usually is a cytosine residue and consequently, the 5' end of the upper strand is usually a guanosine residue.

The RNA transcription product (c) of the C domain functions as a reporter molecule for the presence and quantity of analyte (Figure 3). Signal amplification occurs because each template produces 10¹ to 10⁴ transcripts. The sequence of this domain is designed with a random sequence, evaluated by computer analysis to minimize the possibility of cross-reaction with other probes in the system.

The sequence of the C domain is designed for a specific detection scheme and several such schemes may be employed to quantify the transcripts. For example, the transcription product (c) of the C domain may be subdivided into 2 subdomains - c₁ and c₂ (Figure 3). Subdomain c₁ is complementary to a transcript capture probe which has been immobilized on a solid substrate. Subdomain c₂ is complementary to a labelling probe. After hybridization the amount of label retained is linearly proportional to the amount of analyte present in the original sample.

In an alternate embodiment the transcript of the C domain has only a c₁ subdomain. The C domain is transcribed in the presence of labelled ribonucleotide triphosphates and the labelled transcript is subsequently bound to an immobilized transcript capture probe through its complementary c₁ subdomain and quantified.

In yet another embodiment the transcript of the C domain has only a c₂ subdomain. The C domain is transcribed in the presence of biotinylated ribonucleoside triphosphates and the transcripts are captured on avidin beads. The transcripts are then annealed to a labelling probe through a complementary c₂ subdomain and quantified.

Several other methods of labeling and detecting the transcript of the amplifying probe are possible, including the simultaneous use of labeled ribonucleotides and avidin/biotin coupling, and will be obvious to those skilled in the art.

The B and C domains of polymerase probes may be prepared by cloning, enzymatic assembly, chemical cross-linking techniques, direct chemical synthesis or a combination thereof. When prepared by cloning, nucleic acid sequences that encode the entire B/C domain or fragments thereof can be made in single- or double-stranded form by conventional cloning procedures.

The B domain is double stranded, and the C domain may be either single or double stranded. Double stranded domains can be created in two ways: The strands can be cloned separately and subsequently the complementary strands hybridized; or alternatively, the probe can be cloned as a single stranded, self-annealing polynucleotide (e.g. - b' c' c b). In this case four to ten additional nucleotides, preferably 5 - 7 nucleotides, are added to the sequence as a spacer between c and c' to allow for proper contouring of the double-stranded region when it is self-annealed. The spacer is usually poly-A, but may be modified to minimize hybridization cross-reactivity between various probes in the assay.

The A domain can be conjugated to the B/C domain through interposed linking agents such as nucleic acid, amino acid, carbohydrate or polyol bridges, or through other cross-linking agents. The B/C domain may be synthesized with a 5'-nucleic acid residue that has been derivatized to have a functional group that provides a linking site for the A domain, or the residue can be derivatized after the oligonucleotide has been synthesized to provide such a site. A preferred procedure for chemical cross-linking is to incorporate a N⁴-modified cytosine base at the 5'-end of the polynucleotide as described in the commonly owned E.P.A. Publication No. 0225807.

In a more preferred embodiment the conjugation of the B/C domain DNA to the A domain antibody can be conducted in a manner analogous to enzyme conjugation. The B/C domain is synthesized to contain an alkylamine moiety. The alkylamine is reacted with a heterobifunctional cross linking agent such as succinimidyl 4-(maleimidomethyl)cyclohexane-1-carboxylate (SMCC). After column purification, the DNA can be reacted specifically with sulfhydryl functionalities. An antibody may contain a reactive sulfhydryl (such as in reduced F(ab')₂ or may be modified to contain a sulfhydryl (such as when modified with N-succinimidyl 3-(2-pyridyldithio)propionate (SPDP) then reduced). Direct DNA-amine to antibody-amine conjugation is also possible with homobifunctional reagents, but is typically more difficult to control.

### B - Multimer Probes

Another embodiment of the invention, is a DNA amplification probe (referred to as a "multimer probe") containing two functional domains, a first domain, A, which is the same as domain A of the polymerase probe described supra, and a second (M - Figure 1D) which comprises several repeating subunits. This probe, like the polymerase probe described supra, is also used for signal amplification in immunoassays.

The polynucleotide of the M domain may be a linear or branched polymer of the same repeating single-stranded oligonucleotide subunit or different single-stranded oligonucleotide subunits. These subunits are capable of hybridizing specifically and stably to a single-stranded nucleotide of interest, typically a labeled oligonucleotide or another multimer. These units will normally be 15 to 50, preferably 15 to 30, nucleotides in length and have a GC content in the range of 40% to 60%. The total number of oligonucleotide units in the multimer will usually be in the range of 3 to 50, more usually 10 to 20. The oligonucleotide units of the multimer may be composed of RNA, DNA, modified nucleotides or combinations thereof.

The oligonucleotide subunits of the multimer may be covalently linked directly to each other through phosphodiester bonds or through interposed linking agents such as nucleic acid, amino acid, carbohydrate or polyol bridges, or through other cross-linking agents that are capable of cross-linking nucleic acid or modified nucleic acid strands. The site(s) of linkage may be at the ends of the subunit (in either normal 3'-5' orientation or randomly oriented) and/or at one or more internal nucleotides in the strand.

In linear multimers the individual subunits are linked end-to-end to form a linear polymer. In one type of branched multimer three or more oligonucleotide units emanate from a point of origin to form a branched structure. The point of origin may be another oligonucleotide subunit or a multifunctional molecule to which at least three units can be covalently bound. In another type, there is an oligonucleotide subunit backbone with one or more pendant oligonucleotide subunits. These latter-type multimers are "fork-like", "comb-like" or combination "fork-" and "comb-like" in structure. The pendant units will normally depend from a modified nucleotide or other organic moiety having appropriate functional groups to which oligonucleotides may be conjugated or otherwise attached. See Figure 1D.

The multimer may be totally linear, totally branched, or a combination of linear and branched portions. Preferably there will be at least two branch points in the multimer, more preferably at least 15, preferably 15 to 50. The multimer may include one or more segments of double-stranded sequences.

Domain M may be prepared by cloning (if linear), enzymatic assembly, chemical cross-linking techniques, direct chemical synthesis or a combination thereof. These methods of synthesis are fully disclosed in the commonly owned International patent application, Pub.No. 89/03891, published 5 May 1989. In the case of linear multimers prepared by cloning, nucleic acid sequences that encode the entire multimer or fragments thereof can be made in single- or double-stranded form by conventional cloning procedures. When made in double-stranded form, the multimers/fragments are ultimately denatured to provide single-stranded multimers/fragments. Multimers may be cloned in single-stranded form using conventional single-stranded phage vectors such as M13. Fragments can be linked enzymatically or chemically to form the domain M multimer. When assembled enzymatically, the individual units are ligated with a ligase such as T4 DNA or RNA ligase, as the case may be. When prepared by chemical cross-linking, the individual units may be synthesized with one or more nucleic acids that have been derivatized to have functional groups that provide linking sites or derivatized after the oligonucleotide has been synthesized to provide such sites. A preferred procedure for chemical cross-linking is to incorporate N⁴-modified cytosine bases into the nucleotide as described in commonly owned E.P.A. No. 225 807.

When prepared by direct chemical synthesis oligonucleotides containing derivatized nucleic acids or equivalent multifunctional molecules whose functional groups are blocked are made by conventional oligonucleotide synthesis techniques. The functional groups are unblocked and oligonucleotide units are synthesized out from the unblocked site(s).

A generic structure for the molecules used to generate branch points in the multimers is as follows: 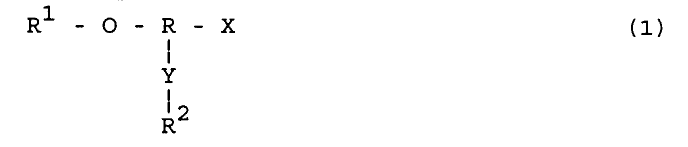 where R is an organic moiety, preferably a nucleic acid, R¹ is a hydroxyl protecting group that can be removed under conditions that do not remove synthetic nucleic acid from a solid phase and do not remove exocyclic nitrogen or phosphate protecting groups, X is a phosphorus-containing group that facilitates nucleic acid synthesis, such as a protected phosphoramidite, phosphonate or phosphate group, Y is a radical derived from a nucleophilic group such as an amino, hydroxyl, sulfhydryl or protected phosphate, and R² is R¹ or a blocking or protective group that can be removed and replaced with hydrogen without affecting R¹. In molecules used to generate bifurcated or "fork-like" branching, R¹ and R² are the same; whereas in molecules used to generate "comb-like" branching, R2 is a blocking group that is stable in the presence of an R¹ deblocking reagent. Figure 4 schematically illustrates the procedures used to synthesize multimers having "comb-like" branches, "fork-like" branches, or combinations thereof.

Part A of Figure 4 depicts a conventional oligonucleotide synthesis scheme for preparing a linear oligonucleotide, such as the automated phosphoramidite method (Warner et al., DNA (1984) 3:401). The dark block represents a solid support, N represents a nucleotide and p-N-OR₁ (R₁ is equivalent to R¹ below), a conventional nucleotide derivative having appropriate protecting groups.

Part B shows the procedure for making a comb-like multimer. The compound : 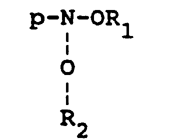 represents a modified base of formula (2) below. An oligomer unit of desired size and sequence is synthesized and left on the support. One or more N⁴-modified cytosine bases are then incorporated into the chain by said automated procedure. Preferably, the modified base has the formula: 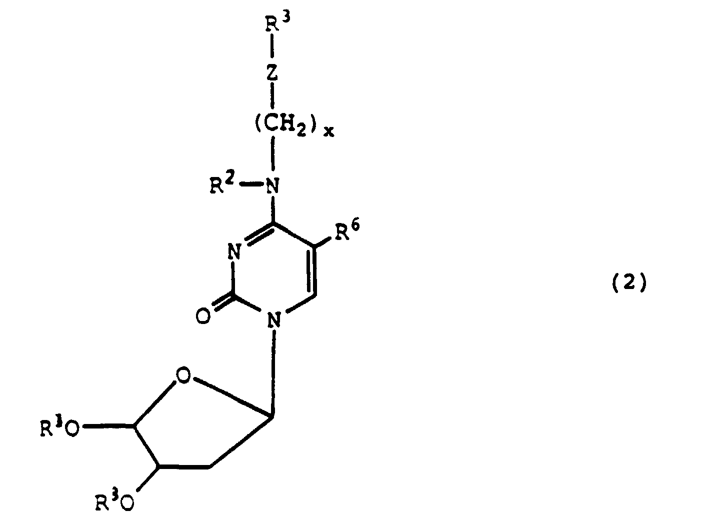 where Z is a nucleophile such as -O-, -NH-, -S-, PO₄=, and 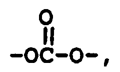 R¹ is a blocking or protective group such as dimethoxytrityl (DMT) or pixyl that is generally base-stable and acid sensitive, R² is hydrogen or methyl, R³ is a blocking or protective group that can be removed and replaced with hydrogen without affecting R¹ such as 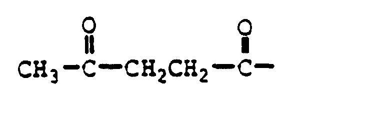 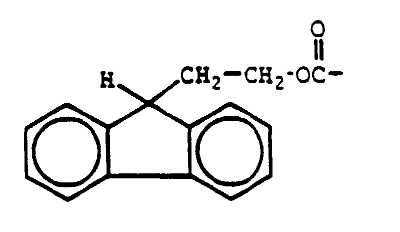 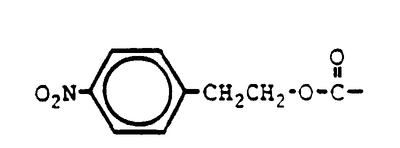 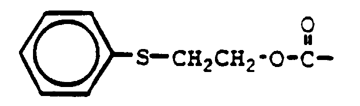 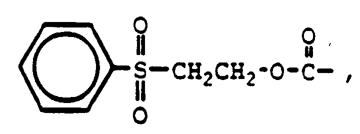 and

CH₃O-CH₂-CH₂-O-CH₂-

R⁵ is a phosphoramidite or other phosphorus derivative that enables addition of nucleotides to the 5' position of an oligonucleotide chain during chemical synthesis (e.g., a phosphodiester, phosphotriester, etc.), R⁶ is methyl, hydrogen, I, Br, or F, and X is an integer in the range of 1 to 8, inclusive. When more than one modified base is incorporated they are preferably spaced by intermediate bases in the chain, most preferably by a -TT- dimer. Additional oligonucleotide units may be incorporated into the backbone followed by additional modified bases and so forth.

The N⁴ nucleophile group is then deprotected (R³ is removed) and additional oligonucleotide units are generated therefrom by the automated procedure. Residual R¹ groups at the chain termini are removed and the branched "comb-like" multimer is cleaved from the support.

Part C of Figure 4 depicts the general procedure for making "fork-like" multimers. Again, an oligomer unit of desired size and sequence is synthesized by conventional techniques and left on the support. A blocked, bifunctional phosphorus-containing group (represented as XP in Part C) such as a blocked phosphoramidite, is then incorporated into the chain by the automated procedure. Preferred bifunctional phosphorus-containing groups are blocked phosphoramidites of the formula 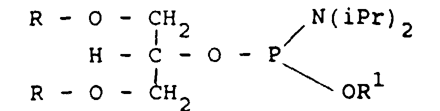 where R is said hydroxyl protecting group, iPr is isopropyl, and R¹ is methyl or beta-cyanoethyl. Most preferably R is DMT and R¹ is beta-cyanoethyl.

Alternatively, the N⁴-modified cytosine base where R₁=R₂ (e.g., DMT) can be used.

The two protecting groups are then removed and additional oligonucleotide units are generated therefrom by the automated procedure. Residual R¹ groups are removed and the bifurcated multimer is cleaved from the support.

Parts D and E depict procedures where two or more bifurcated multimers, "comb-like" multimers or combinations thereof are spliced together enzymatically or chemically. Generally, the bifurcated and/or "comb-like" multimers are prepared as above and removed from the support. They are then combined in solution using the enzymatic or chemical linkage procedures described above.

Part F shows the procedure for synthesizing a multiple "comb-like" multimer. This procedure is a variation of the procedure shown in Part B and involves incorporating modified bases in the dependent side chains and generating secondary oligonucleotide side chains therefrom.

Suitable cleavable linker molecules may be incorporated into the multimers at predetermined sites for the purpose of analyzing the structure of the multimer or as a means for releasing predetermined segments (such as the portion of the multimer that binds to the labeled oligonucleotide). Subsequent to multimer synthesis and purification these linkers can be cleaved specifically without additional degradation of the nucleotide structure of the multimer. A preferred type of linker molecule was designed to contain a 1,2-diol group (which can be cleaved selectively by periodates) as well as a protected hydroxyl and phosphoramidite derived hydroxyl group to permit the linker to be incorporated into any DNA fragment by standard phosphoramidite chemistry protocols. A preferred embodiment of such a linker is the compound: 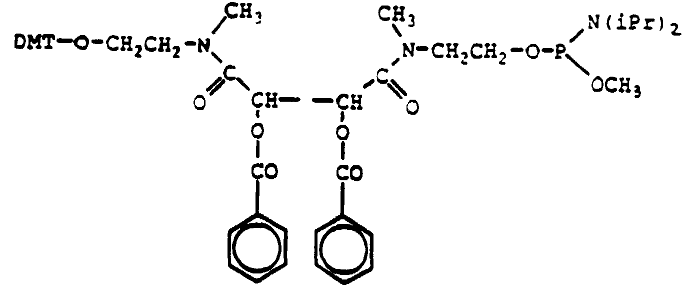 where DMT and iPr are as defined previously. After incorporation into a DNA fragment and complete deprotection the linker-containing fragment has the following structure: 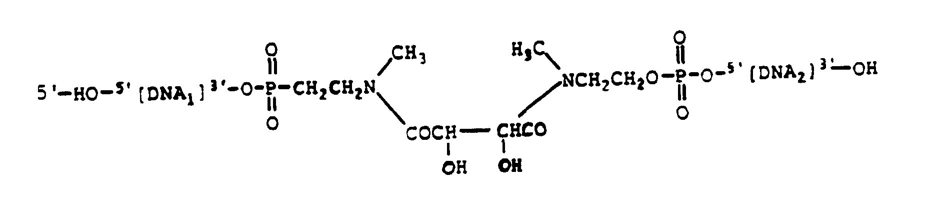 where DNA₁ and DNA₂ represent DNA subfragments which may be the same or different. Reaction of this fragment with sodium periodate cleaves the fragment into the following subfragments:

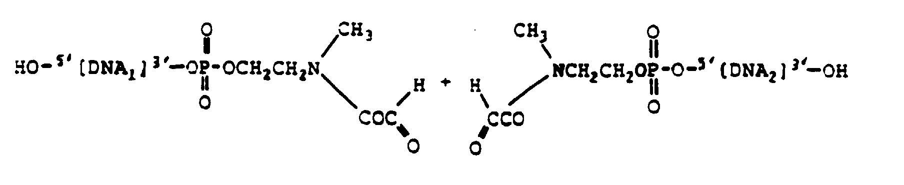

Alternatively, the 1,2-diol group may be replaced with linker groups that contain a hydroxylamine-sensitive linkage, a base-sensitive sulfone linkage, or a thiol-sensitive disulfide linkage. Such linker groups may be derived from conventional cross-linking agents that are used to conjugate proteins to other entities. Likewise, protecting groups other than DMT may be used.

The functional aspects of the polymerase probe may be combined with the multimer probe structure by synthesizing a multimer probe having a M domain with subunits comprising multiples of the promoter/template (B/C) domains of the polymerase probe. Like multimer probes the multimeric subunits may be either in a linear array or branched molecules.

Like polymerase probes, the A domain of multimer probes can be conjugated to the M domain through interposed linking agents such as nucleic acid, amino acid, carbohydrate or polyol bridges, or through other cross-linking agents. The M domain may be synthesized with a 5'-nucleic acid residue that has been derivatized to have a functional group that provides a linking site for the A domain, or the residue can be derivatized after the oligonucleotide has been synthesized to provide such a site. A preferred procedure for chemical cross-linking is to incorporate a N⁴-modified cytosine base at the 5'-end of the polynucleotide as described in the commonly owned E.P.A. publication No. 0225807 and in Section I(A), supra.

### II - Immunoassays

In another aspect, the invention provides immunoassays employing hybrid amplifier probes to detect and quantify the presence and concentration of an immunogenic analyte. The analyte may be any known immunogen of interest. The analyte may be present in low concentration in a prepared sample or it may be a minority species in a heterogeneous mixture of biological material. The analyte may be present in a variety of sources, e.g., biological fluids or tissues, food stuffs, environmental materials, etc., or it may be synthesized in vitro.

In a first step, the sample containing the analyte is prepared by any of a variety of methods known to those skilled in the art.

Amplifier probes can be employed to detect an analyte in any number of immunoassay protocols - conducted either in solution or immobilized upon a solid phase. If the immunoassay is conducted in solution, the analyte is either bound to the amplifier probe directly or indirectly through one or more antibodies specific for the analyte. Polypeptide/oligonucleotide hybrid amplifier probes of the invention can be utilized in this fashion in most soluble immunoassay protocols. See, for example, U.S. Patent No. 4,778,751.

In an immunoassay utilizing a solid phase, the analyte (which can either be an antigen or an antibody) is either immobilized directly upon a solid phase; indirectly by binding the analyte to a first antibody that has first been bound to a solid phase; or by a sandwich assay (Figure 2A) in which a second antibody recognizes the analyte and in turn is recognized by the first antibody bound to the solid phase.

If the analyte is to be directly bound to a solid phase, the sample containing analyte is contacted with a nitrocellulose filter or similar means for non-selectively binding protein. If the analyte is to be indirectly and selectively bound to a solid phase a first antibody, directed to an epitope of the analyte or to a second antibody, is conjugated to the solid phase and subsequently used to bind the analyte or a second antibody bound to the analyte.

An additional antibody, directed to a second epitope of the analyte can then be employed as a linker between the analyte and an amplifier probe. The amplifier probe, either a polymerase probe or a multimer probe, whose A domain is directed to the second antibody, can then be conjugated to the linker antibody, forming a string consisting of the first antibody bound to a solid phase, a second antibody, the analyte, a linker antibody, and the amplifier probe (Figure 2A).

Suitable binding and hybridization conditions for an immunoassay of the invention are as follows.

In a preferred embodiment, the analyte is indirectly immobilized upon a solid phase, for example a 96-well polyvinyl chloride (PVC) plate. In the sandwich assay depicted in Figure 2A, 50µl of the first antibody (anti-rabbit at a concentration of 20µg/ml in 0.2M NaHCO₃) is added to a well. The well is covered and incubated for 2 hours at room temperature in a humidified atmosphere. The solution is then aspirated from the well. The well is then washed twice with a blocking buffer comprising 3% bovine serum albumin in phosphate buffered saline solution containing 0.02% sodium azide. The well is next incubated with blocking buffer for 20 minutes at room temperature in a humidified atmosphere and then washed twice more with blocking buffer.

50µl of the second antibody (rabbit-anti analyte also at a concentration of 20µg/ml) is added to the well and the procedure described above is repeated. Next a series of dilutions, in blocking buffer, of the analyte is prepared. 50µl of each dilution is added to a well. The plate is covered and incubated for 2 hours at room temperature in a humidified atmosphere. Following this incubation the wells are washed four times with blocking buffer. The third antibody (mouse-anti-analyte) is next added in the same fashion, followed by addition of the hybrid amplifier probe. Since occasional tissue preparations may contain anti-DNA antibodies, the blocking buffer used for the washes which follow the addition of the probe also contains 20µg/ml salmon sperm DNA.

When hybrid multimer probes are used, a nucleic acid hybridization step follows the complexing of the analyte and antibodies. Usually, hybridization conditions consist of an aqueous medium, particularly a buffered aqueous medium, which includes various additives. These additives can include the polynucleotides to be hybridized, salts (e.g. sodium citrate 0.017M to 0.17M and/or sodium chloride 0.17M to 1.7M), non-ionic detergents such as NP-40 or Triton™ X-100 (0.1 to 1.0%) which do not interfere with the antigen/antibody complex, and carrier nucleic acids. The mixture is incubated for 15 to 75 minutes at 35°C to 55°C.

If the conditions employed for hybridization of the labeled oligonucleotide to the multimer subunits causes instability in the antigen/antibody complexes, the hybridization step can be preceded by treatment of the complex with a protein crosslinker such as glutaraldehyde, or a similar method of stabilizing the complex. Once the protein complex is stabilized, conditions of nucleic acid hybridization may be altered to include SDS (up to 1%); nonaqueous solvents such as dimethylformamide, dimethylsulfoxide, and formamide; and temperatures up to 70°C.

It is important to adjust nucleic acid hybridization conditions to maintain the stability of the antigen/antibody complexes. For example, the hybridization of shorter stretches of complementary sequences can be accomplished at lower temperatures. In a preferred embodiment, complementary single-stranded regions are 12 to 20 bases. In a preferred embodiment, the hybridization temperature is 35 to 45 degrees C. Furthermore, since the only single-stranded oligonucleotides present at this step are the complementary strands, higher stringency and consequently lower salt concentrations (0.3M Na) can be tolerated.

### 1 - Multimer Probe

If a multimer probe is employed, labeled oligonucleotide which is substantially complementary to the repeating polynucleotide of the M domain is subsequently added under conditions which permit it to hybridize to the complementary oligonucleotide units of the multimer. The resulting solid phase labeled nucleic acid complex is then separated from excess labeled oligonucleotide, by washing to remove unbound labeled oligonucleotide, and read.

The amplification may be multiplied by the use of more than one species of multimer (as defined by subunit sequence) in the assay. In such instances a second multimer is designed to bind to the repeat sequence of the first multimer and the complex is subsequently labeled with an oligonucleotide that is substantially complementary to the repeat sequence of the second multimer. Any number of multimers may be bound in series in this manner to achieve even greater amplification.

### 2 - Polymerase Probe

If a polymerase probe is employed, the RNA polymerase specific for the promoter region (domain B) of the amplifier probe is next added under appropriate transcription conditions and multiple RNA copies (c) of the C domain template (c') are produced. The amount of transcript is proportional to the quantity of the analyte in the initial preparation.

Transcription conditions consist of an aqueous medium, preferably a buffered aqueous medium, with appropriate salts, usually including a magnesium salt, rATP, rUTP, rGTP, rCTP, a RNA polymerase enzyme and usually include various denaturing agents, protein carriers, and RNAse inhibitors. Incubation is usually for 15 to 90 minutes, usually 60 minutes; and at a temperature which is optimal for the chosen enzyme, usually 35°C to 42°C, usually 37°C.

The sequence of the C domain is designed for a specific detection scheme and several such schemes may be employed to quantify the transcripts. For example, the transcription product (c) of the C domain may be subdivided into 2 subdomains - c₁ and c₂ (Figure 2B). Subdomain c₁ is complementary to a transcript capture probe which has been immobilized on a solid substrate. Subdomain c₂ is complementary to a labelling probe. After hybridization the amount of label retained is linearly proportional to the amount of analyte present in the original sample.

In an alternate embodiment the transcript of the C domain has only a c₁ subdomain. The C domain is transcribed in the presence of labelled ribonucleotide triphosphates and the labelled transcript is subsequently bound to an immobilized transcript capture probe through its complementary c₁ subdomain and quantified.

In yet another embodiment the transcript of the C domain has only a c₂ subdomain. The C domain is transcribed in the presence of biotinylated ribonucleoside triphosphates and the transcripts is captured on avidin beads. The transcript is then annealed to a labelling probe through its complementary c₂ subdomain and quantified.

Several other methods of labeling and detecting the transcript of the amplifying probe are possible, including the simultaneous use of labeled ribonucleotides and avidin/biotin coupling, and will be obvious to those skilled in the art.

### 3 - General Considerations

The solid phase that is used in the assay may be particulate or be the solid wall surface of any of a variety of containers, e.g., centrifugal tubes, columns, microtiter plate wells, filters, tubing, etc. Preferably, particles will be employed of a size in the range of about 0.4 to 200 microns, more usually from about 0.8 to 4.0 microns. The particles may be any convenient material, such as latex, or glass. The first antibody specific for the analyte, or for a second antibody which is specific for the analyte, may be stably attached to the solid surface through functional groups by known procedures.

The labeling probes will, if polymerase probes are employed, include sequences complementary to the c₂ subdomain of the transcripts of the amplifier probe; and if multimer probes are employed, labeling probes will include sequences complementary to the repeating units of the B domain. The labelling probe will include one or more molecules ("labels"), which directly or indirectly provide for a detectable signal. The labels may be bound to individual members of the complementary sequence or may be present as a terminal member or terminal tail having a plurality of labels. Various means for providing labels bound to the sequence have been reported in the literature. See, for example, Urdea et al, Nucl. Acids Res. (1988) 16:4937, Leary et al., Proc. Natl. Acad. Sci. USA (1983) 80:4045; Renz and Kurz, Nucl. Acids Res. (1984) 12:3435; Richardson and Gumport, Nucl. Acids Res. (1983) 11:6167; Smith et al., Nucl. Acids Res. (1985) 13:2399; Meinkoth and Wahl, Anal. Biochem. (1984) 138:267. The labels may be bound either covalently or non-covalently to the complementary sequence. Labels which may be employed include radionuclides, fluorescers, chemiluminescers, dyes, enzymes, enzyme substrates, enzyme cofactors, enzyme inhibitors, enzyme subunits, metal ions, and the like. Illustrative specific labels include fluorescein, rhodamine, Texas red, phycoerythrin, umbelliferone, luminol, NADPH, galactosidase, horseradish peroxidase, etc. See Urdea et al for a comparison of non-radioisotopic labelling methods.

The labelling probes can be conveniently prepared by chemical synthesis such as that described in commonly owned E.P.A. Pub. No. 225807. By providing for a terminal group which has a convenient functionality, various labels may be joined through the functionality. Thus, one can provide for a carboxy, thiol, amine, hydrazine or other functionality to which the various labels may be joined without detrimentally affecting duplex formation with the sequence. As already indicated, one can have a molecule with a plurality of labels joined to the sequence complementary to the labeling sequence. Alternatively, one may have a ligand bound to the labeling sequence and use a labeled receptor for binding to the ligand to provide the labeled analyte complex.

Depending upon the nature of the label, various techniques can be employed for detecting the presence of the label. For fluorescers, a large number of different fluorometers are available. With enzymes, either a fluorescent or a colored product can be provided and determined fluorometrically, spectrophotometrically or visually. The various labels which have been employed in immunoassays and the techniques applicable to immunoassays can be employed with the subject assays.

The procedure used in the separation steps of the assay will vary depending upon the nature of the solid phase. For particles, centrifugation or filtration will provide for separation of the particles, discarding the supernatant or isolating the supernatant. Where the particles are assayed, the particles will be washed thoroughly, usually from one to five times, with an appropriate buffered medium containing detergent, e.g., PBS with SDS. When the separation means is a wall or support, the supernatant may be isolated or discarded and the wall washed in the same manner as indicated for the particles.

### 4. Competitive Immunoassays

Hybrid amplifier probes of the subject invention can also be employed as a means for labeling antigen in a competitive immunoassay. In the typical assay, excess antibody, polyclonal or monoclonal, preferably monoclonal, which specifically binds to the analyte, is immobilized upon a solid phase, usually in a multiple well plate. A standard concentration of labeled antigen is mixed with serial dilutions of a sample thought to contain an unknown amount of analyte. The separate dilutions are then exposed to the immobilized antibody and the amount of bound label is measured. The ability of the unlabeled analyte to compete with the labeled analyte is used to calculate the amount of analyte in the sample.

In one embodiment, a B/C domain polynucleotide moiety of the polymerase probe is conjugated to a competitive immunogen. This hybrid molecule is then used as the labeled antigen described supra. After the competitive binding, polymerase (specific for the B domain promoter) is added and the transcription product is assayed as described in Section I(A), supra.

In another embodiment, a M domain polynucleotide moiety of the multimer probe is conjugated to a competitive immunogen. This hybrid molecule is then used as the labeled antigen described supra. After the competitive binding, the M domain is labeled and quantified as described, supra.

For immunogen coupling to DNA, homobifunctional reagents such as disuccinimidyl suberate (DSS), ethylene glycol bis(succinimidylsuccinate) (EGS), or p-phenylene diisothiocyanate (DITC), are probably more useful coupling methods than those described in Section I(A), supra.

Kits for carrying out amplified immunoassays according to the invention will comprise, in packaged combination, the following reagents: a solid phase that is either capable of binding the antigen analyte or alternatively already has a linking first antibody bound to it if the assay format is one in which the analyte is indirectly bound to the solid phase through a bound first antibody or through a second antibody; optionally linker second and third antibodies which are specific for the analyte, and a hybrid linker probe if the assay format is one in which a polynucleotide amplifier probe is used; the amplifier probe; the appropriate DNA-directed RNA polymerase and immobilized transcription capture probes (if a polymerase amplifier probe is used); and an appropriate labelling probe; These reagents will typically be in separate containers in the kit. The kit may also include hybridization buffers, wash solutions, negative and positive controls and written instructions for carrying out the assay.

The following examples are presented as illustrations of the present invention.

### Example 1 - Immunoassay for the Presence of an HCV Antigen in Human Serum

This assay is designed to test directly for the presence of the C-100 antigen of the Hepatitis C virus. The general protocol for this immunoassay is as described, supra. Microtiter plates are prepared using goat-anti-mouse as the first antibody. This antibody is immobilized in the wells of a 96-well microtiter plate. The second antibody is a mouse monoclonal antibody specific for an epitope of HCV C-100. Duplicate dilutions of serum from individuals to be screened for HCV are prepared in blocking buffer and incubated in the microtiter wells along with appropriate non-infected controls. The third antibody is a combination of polyclonal rabbit-antibodies which recognizes each of the HCV antigens. The final antibody is a hybrid T7 polymerase amplifier probe. The domain A polypeptide moiety functions as a goat-anti-rabbit antibody and completes the immunoassay "sandwich".

After addition of the T7 polymerase probe, transcription of domain C is effected by incubating the complex in 20 µl of a solution containing 40 mM Tris HCl (pH 8), 20 mM MgCl₂, 10 mM NaCl, lmM Spermidine, 10 mM Dithiothreitol, 0.15 mg/ml Bovine Serum Albumin, 1.25 mM each of rATP, rCTP, rGTP, rUTP, 1600 units/ml RNasin, and 2000 units/ml T7 RNA polymerase. This mixture is incubated at 37° C for 1 hour. Transcription is terminated by addition of 20 µl of a solution containing 8 X SSC, and 0.2% SDS and the entire mixture is transferred to new wells containing an immobilized capture probe with c₁' sequences. Capture of the domain C transcripts (Figure 3) is effected by incubation at 55° C for 1 hour followed by two washes with 0.1 X SSC, 0.1% SDS.

The domain C transcripts are then labelled by addition of 50 fmol of enzyme-labeled probe (c₂') in 40 µl of 4 X SSC, 100 µg/ml poly A for 15 min. at 55°C. Finally, the complex was washed twice with 0.1 X SSC, 0.1% SDS, followed by two washes with 0.1X SSC.

For AP detection, an enzyme-triggered dioxetane reaction (Schapp et al. (1987) Tet. Lett. 28: 1159-1162 and US Patent 4,857,652), obtained from Lumigen Inc., is employed. The detection procedure is as follows. For the labeling step 20 µl HM buffer with the AP probe is added to each well and the wells are incubated at 55°C for 15 min. The supernatant is removed and the wells are washed 2 x with 380 µl of 0.1 x SSC and 0.1% SDS. The wells are then washed 2 x with 380 µl of 0.1 x SSC to remove any remaining SDS. 20 µl of 3.3 x 10⁻⁴ M dioxetane in CTAB buffer is added to each well. The wells are tapped lightly so that the reagent falls to the bottom and gently swirled to distribute the reagent evenly over the bottom. The wells are covered with the microtiter plate sealer and incubated in a 37°C oven for one hour. The wells are then read with a luminometer, and quantified relative to a standard curve generated with known amounts of antigen.

### Example 2 - Immunoassay for the Presence of Antibody to HCV in Human Serum

Microtiter plates are first coated with the HCV C-100 antigen from the Hepatitis C virus. A solution containing coating buffer (50mM Na Borate, pH 9.0), 21 ml/plate, BSA (25µg/ml), HCV C-100 (2.50µg/ml) is prepared just prior to addition. After mixing for 5 minutes, 0.2ml/well of the solution is added to the plates, they are covered and incubated for 2 hours at 37°C, after which the solution is removed by aspiration. The wells are washed once with 0.4ml wash buffer (100mM Na phosphate,pH 7.4, 140mM NaCl, 0.1% casein, 1% (w/v) Triton X-100, 0.01% (w/v) Hibitane)™. After removal of the wash solution, 200 µl/well of Postcoat™ solution (10mM Na phosphate, pH7.2, 150mM NaCl, 0.1% (w/v) casein, 3% sucrose and 2mM phenylmethylsulfonylfluoride (PMSF), is added, the plates are loosely covered to prevent evaporation, and are allowed to stand at room temperature for 30 minutes. The wells are then aspirated to remove solution, and lyophilized dry overnight, without shelf heating.

To perform the immunoassay, 20µl of duplicate dilutions of serum samples or controls are added to wells containing 200µl of sample diluent (100 mM Na phosphate, pH 7.4, 500mM NaCl, 1mM EDTA, 0.1% (w/v) casein, 0.01% (w/v) Hibitane, 1% (w/v) Triton X-100, 100µg/ml yeast extract). The plates are sealed, and incubated at 37°C for 2 hours, after which the solution is removed by aspiration, and the wells are washed 3 times with 400 µl of wash buffer (PBS containing 0.05% Tween 20).

Subsequent to the addition of serum samples, the wells are treated as in Example 1, above, except that rabbit-anti-human serum is substituted for the third antibody (rabbit-anti-analyte).

### Example 3 - Use of Multimer Probes in the Detection of HCV Infection

In either of the two examples above, multimer probes can be substituted for polymerase probes. All steps are the same in each example up through the addition of amplifier probe, except of course that a multimer probe is used rather than a polymerase probe. Subsequent to the binding of the amplifier probe, a solution of with 50fmol of a labeled oligonucleotide sequence with substantial homology to the multimeric subunit of the M domain is added. This enzyme-labeled probe is added in 40 µl of 4 X SSC, 100 µg/ml poly A for 15 min. at 55°C. Finally, the complex was washed twice with 0.1 X SSC, 0.1% SDS, followed by two washes with 0.1X SSC.

For AP detection, an enzyme-triggered dioxetane reaction (Schapp et al. (1987) Tet. Lett. 28: 1159-1162 and US Patent 4,857,652), obtained from Lumigen Inc., is employed. The detection procedure is as follows. For the labeling step 20 µl HM buffer with the AP probe is added to each well and the wells are incubated at 55°C for 15 min. The supernatant is removed and the wells are washed 2 x with 380 µl of 0.1 x SSC and 0.1% SDS. The wells are then washed 2 x with 380 µl of 0.1 x SSC to remove any remaining SDS. 20 µl of 3.3 x 10⁻⁴ M dioxetane in CTAB buffer is added to each well. The wells are tapped lightly so that the reagent falls to the bottom and gently swirled to distribute the reagent evenly over the bottom. The wells are covered with the microtiter plate sealer and incubated in a 37°C oven for one hour. The wells are then read with a luminometer, and quantified relative to a standard curve generated with known amounts of antigen.

### Example 4 - Use of Polynucleotide Multimer Probe in the Detection of Immobilized Human IgG

A comb-like branch polynucleotide having 15 dependent side chains each containing three sites (total of 45 sites) complementary to a labeled probe and a terminal biotin molecule attached to the backbone was prepared as described above.

Human IgG at various concentrations was immobilized on a solid support. The immobilized IgG was incubated with biotinylated affinity purified goat anti-human IgG (biotin concentration 40 ng) and excess antiserum was washed from the support. The support was then incubated with 200 ng avidin-D and then with 100 fm or 500 fm of the comb-like branched polynucleotide, with appropirate washings. The support was then incubated with horseradish peroxidase labeled oligonucleotide probe complementary to the labeled probe binding sites on the polynucleotide. Optical density (O.D.) readings were made at 492/620 nm.

For comparison purposes, assays were run as above using a polynucleotide with only one labeled probe binding site.

The table below presents the results of these tests.

| O.D. | | | | |
|---|---|---|---|---|
| Human IgG Conc. (ng/ml) | 45 site @ 100 fm | 1 site @ 100 fm | 45 site @ 500 fm | 1 site @ 500 fm |
| 100 | 1.116 | 0.005 | 2.505 | 0.002 |
| 50 | 1.089 | 0.002 | 1.564 | 0.001 |
| 25 | 0.432 | 0.001 | 1.295 | 0.001 |
| 12.5 | 0.303 | 0.001 | 0.451 | 0 |

As indicated above, the 45 site polynucleotide provided a much higher signal than the single site polynucleotide.

## Claims (Claims for the following Contracting State(s): AT, BE, CH, LI, DE, DK, FR, GB, GR, IT, LU, NL, SE)

1. A molecular probe for use as a signal amplifier in immunoassays comprising:
(a) a first domain (A) which is a polypeptide and functions as an antibody specific for a known antigen;
(b) a second domain (B) which is a double-stranded polynucleotide capable of functioning as a promoter for a DNA-dependent RNA polymerase enzyme activity; and
(c) a third domain (C) which is either single- or double-stranded and adjacent to the second domain, such that the third domain is capable of functioning as a template for the promoter activity of the second domain.

2. A probe as claimed in claim 1 in which said second domain B provides a promoter for DNA-dependent RNA polymerase activity derived from the bacteriophage T7.

3. A probe as claimed in claim 1 or claim 2 in which the second domain B is at least 10 and no more than 40 nucleotides long.

4. A probe as claimed in any one of claims 1 to 3 in which the third domain C is at least 30 and no more than 80 nucleotides long.

5. A probe as claimed in claim 2 in which the sequence for the second domain B comprises the sequence:

5'-TAA TAC GAC TCA CTA TA-3'

3'-ATT ATG CTG AGT GAT AT-5'

6. A probe as claimed in claim 2 in which the nucleotide sequence of the second domain B is:

5'-CTG GCT TAT CGA AAT TAA TAC GAC TCA CTA TA-3'

3'-GAC CGA ATA GCT TTA ATT ATG CTG AGT GAT AT-5'

7. A probe as claimed in any one of claims 1 to 6 in which the 5' residue of the upper strand of the nucleotide sequence for the third domain C is adjacent to the second domain B, and is a guanosine residue.

8. A probe as claimed in any one of claims 1 to 7 in which the sequence for the third domain C is:

5′-GGG AGA TGT GGT TGT CGT ACT TAG CGA AAT ACT GTC CGA GTC
G-3′

3'-CCC TCT ACA CCA ACA GCA TGA ATC GCT TTA TGA CAG GCT CAG
C-5'

9. A probe as claimed in claim 8 in which the 3' end of the upper strand of the DNA nucleotide sequence of the second domain B is attached to the 5' end of the upper strand of the nucleotide sequence of domain C.

10. A probe as claimed in claim 1 in which the transcript of the third domain C has two subdomains:
(a) a first subdomain, c₁, which is capable of hybridizing to an oligonucleotide capture linker, the capture linker being capable of hybridizing to a polynucleotide immobilized on a solid substrate; and
(b) a second subdomain, c₂, which is capable of binding to an oligonucleotide label linker, the label linker capable of binding to a quantifiable probe.

11. A probe as claimed in claim 1 in which a functional unit comprising the second and third domains, B and C, is present in multiple repeating units.

12. A method of amplifying a detectable signal in an immunoassay which comprises:
(a) directly or indirectly binding to an analyte a biochemical molecular probe comprising a binding domain (A) which is a polypeptide, a second domain (B) which is a double stranded DNA sequence capable of functioning as a promoter for a DNA-dependent RNA polymerase enzyme activity, and a third domain (C) which is either single- or double- stranded and adjacent to the second domain B, such that the third domain is capable of functioning as a template for the promoter activity of the second domain;
(b) removing unbound probe;
(c) transcribing multiple copies of RNA oligomers which are complementary to the template sequence, c', of the third domain, C, of the probe construct via a DNA-dependent RNA polymerase activity; and
(d) quantifying the RNA transcripts.

13. A method as claimed in claim 12 in which the analyte is first immobilized, directly or indirectly, upon a solid substrate.

14. A method as claimed in claim 12 or claim 13 in which domain A is a polypeptide capable of functioning as an antibody, and the probe is bound directly or indirectly to the analyte through domain A.

15. A method as claimed in claim 14 which employs a probe according to any one of claims 2 to 11.

16. A method as claimed in any one of claims 12 to 15 in which the second domain, B, provides a promoter for the DNA-dependent RNA polymerase of the bacteriophage T7.

17. A method as claimed in any one of claims 12 to 16 in which:
(a) the third domain, C, of said probe is transcribed in the presence of labeled ribonucleotide triphosphates;
(b) the transcripts of the third domain have a first subdomain, c₁, complementary to an oligonucleotide capture probe which is immobilized on a solid substrate; and
(c) said transcripts are immobilized and quantified.

18. A method as claimed in any one of claims 12 to 16 in which:
(a) the third domain, C, of said probe is transcribed in the presence of biotinylated ribonucleotide triphosphates;
(b) the transcript of the third domain has a first subdomain, c₂, which is complementary to a labeled oligonucleotide probe;
(c) the transcripts are immobilized upon an avidinylated solid substrate; and
(d) the transcripts are quantified.

19. A method as claimed in any one of claims 12 to 16 in which:
(a) the third domain, C, of the probe is transcribed in the presence of both labeled and biotinylated ribonucleotide triphosphates;
(b) the transcripts are immobilized upon an avidinylated solid substrate; and
(c) the transcripts are quantified.

20. A method as claimed in any one of claims 12 to 16 in which:
(a) the transcript of the third domain, C, of said probe has two subdomains:
(i) a first subdomain, c₁, which is complementary to an oligonucleotide capture probe, the capture probe being immobilized on a solid substrate; and
(ii) a second subdomain, c₂, which is complementary to a labeled oligonucleotide probe;
(b) the transcript is hybridized to the capture probe;
(c) the labeled probe is hybridized to said transcript; and
(d) the labeled probe is quantified.

21. A method as claimed in any one of claims 12 to 20 wherein a probe is employed in which a unit comprising the second and third domains, B and C, is present in multiple repeating units.

22. A molecular probe for use as a signal amplifier in immunoassays comprising:
(a) a first domain (A) comprising a polypeptide capable of functioning as an antibody which binds specifically to a known analyte; and
(b) a second domain (M) comprising a multiplicity of single-stranded oligonucleotide subunits that are capable of binding specifically to a single-stranded nucleic acid sequence of interest, said multiplicity of single-stranded oligonucleotide subunits being covalently linked to each other.

23. A probe as claimed in claim 22 in which each oligonucleotide unit of the second domain, M, comprises about 15 to 50 bases and has a GC content in the range of about 40% to 60%.

24. A probe as claimed in claim 22 or claim 23 in which the second domain has a linear structure.

25. A probe as claimed in claim 22 or claim 23 in which the second domain has a branched structure.

26. A probe as claimed in claim 22 or claim 23 wherein at least a portion of the oligonucleotide subunits of the second domain are linked via a multifunctional moiety derived from a compound of the formula: 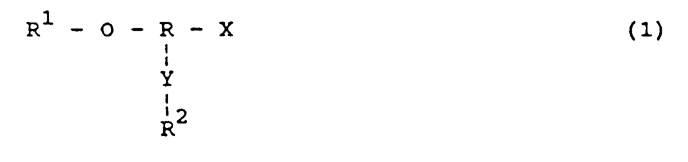 where R is an organic moiety, preferably a nucleic acid, R¹ is a hydroxyl protecting group that can be removed under conditions that do not remove synthetic nucleic acid from a solid phase and do not remove exocyclic nitrogen or phosphate protecting groups, X is a phosphorus-containing group that facilitates nucleic acid synthesis, Y is a radical derived from a nucleophilic group, and R² is R¹ or a blocking or protective group that can be removed and replaced with hydrogen without affecting R¹.

27. A probe as claimed in claim 22 or claim 23 wherein at least a portion of the oligonucleotide subunits are linked via a multifunctional moiety derived from a compound of the formula: 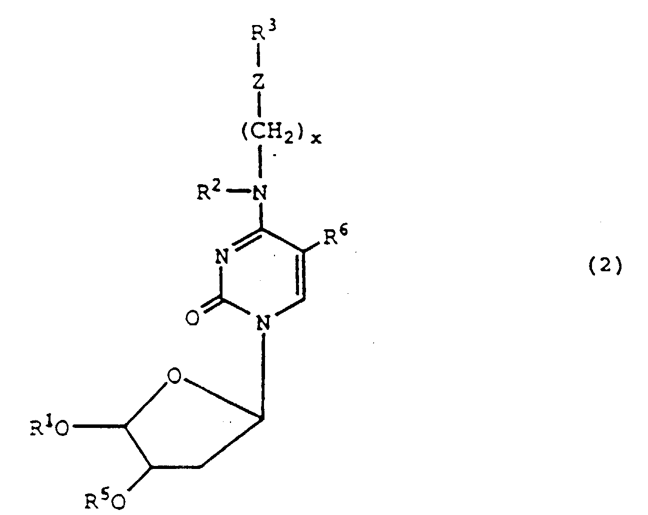 where Z is a nucleophile, R¹ is a protective group that is generally base-stable and acid sensitive, R² is hydrogen or methyl, R³ is a protective group that can be removed and replaced with hydrogen without affecting R¹, R⁵ is a phosphorus derivative that enables addition of nucleotides to the 5' position of an oligonucleotide chain during chemical synthesis, R⁶ is methyl, hydrogen, I, Br, or F, and X is an integer in the range of 1 to 8, inclusive.

28. A probe as claimed in claim 22 or claim 23 wherein at least a portion of the oligonucleotide subunits are linked via a multifunctional moiety derived from a compound of the formula: 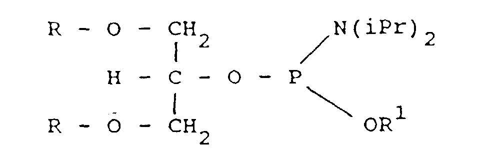 where R is said hydroxyl protecting group, iPr is isopropyl, and R¹ is methyl or beta-cyanoethyl.

29. A method of amplifying a detectable signal used to detect and quantify an antigen in an immunoassay which comprises:
(a) directly or indirectly binding to an analyte a biochemical molecular probe comprising a binding domain (A) which is a polypeptide covalently attached to a second domain (M) which comprises a multiplicity of single-stranded DNA oligomeric subunits capable of hybridizing to a single-stranded labeled oligonucleotide, said multiplicity of single-stranded oligonucleotide subunits being covalently linked to each other;
(b) removing unbound probe;
(c) hybridizing single-stranded labeled oligonucleotides, which comprise a nucleotide sequence which is substantially complementary to the subunit sequence of the domain M probe, to the subunits of the second domain;
(d) removing unbound labeled oligonucleotide; and
(e) quantifying the amount of labeled oligonucleotide bound to the probe.

30. A method as claimed in claim 29 in which the analyte is first immobilized, directly or indirectly, on a solid substrate.

31. A method as claimed in claim 29 or claim 30 in which domain A is a polypeptide capable of functioning as an antibody, and the probe is bound directly or indirectly to the analyte via domain A.

32. A method as claimed in any one of claims 29 to 31 in which the polypeptide of domain A specifically binds to an epitope of an antibody which specifically binds to the analyte.

33. A method as claimed in claim 31 wherein a probe according to any one of claims 23 to 28 is employed.

34. A method of amplifying a detectable signal in a competitive immunoassay which comprises:
(a) providing a molecular probe comprising:
(i) a first domain (A) which is capable of functioning as an immunogen;
(ii) a second domain (B) which is a double-stranded polynucleotide capable of functioning as a promoter for a DNA-dependent RNA polymerase enzyme activity; and
(iii) a third domain (C) which is either single- or double-stranded and adjacent to the second domain, such that the third domain is capable of functioning as a template for the promoter activity of the second domain;
(b) immobilizing the probe, directly or indirectly, upon a solid substrate in the presence of a competing analyte;
(c) removing unbound probe;
(d) transcribing multiple copies of RNA oligomers which are complementary to the template sequence, c', of the third domain, c, of the probe construct via a DNA-dependent RNA polymerase activity;
and
(e) quantifying the RNA transcripts.

35. A method of amplifying a detectable signal in a competitive immunoassay which comprises:
(a) providing a molecular probe comprising:
(i) a first domain (A) which is capable of functioning as an immunogen and is not an oligonucleotide; and
(ii) a second domain (M) comprising a multiplicity of single-stranded oligonucleotide subunits that are capable of binding specifically to a single-stranded nucleic acid sequence of interest.
(b) immobilizing the probe, directly or indirectly, upon a solid substrate in the presence of a competing analyte;
(c) removing unbound probe;
(d) hybridizing single-stranded labeled oligonucleotides, which comprise a nucleotide sequence which is substantially complementary to the subunit sequence of the domain M probe, to the subunits of the second domain;
(e) removing unbound labeled oligonucleotide; and
(f) quantifying the amount of labeled oligonucleotide bound to the probe.

## Claims (Claims for the following Contracting State(s): ES)

1. A method of amplifying a detectable signal in an immunoassay which comprises:
(a) directly or indirectly binding to an analyte a biochemical molecular probe comprising a binding domain (A) which is a polypeptide, a second domain (B) which is a double stranded DNA sequence capable of functioning as a promoter for a DNA-dependent RNA polymerase enzyme activity, and a third domain (C) which is either single- or double- stranded and adjacent to the second domain B, such that the third domain is capable of functioning as a template for the promoter activity of the second domain;
(b) removing unbound probe;
(c) transcribing multiple copies of RNA oligomers which are complementary to the template sequence, c', of the third domain, C, of the probe construct via a DNA-dependent RNA polymerase activity; and
(d) quantifying the RNA transcripts.

2. A method as claimed in claim 1 in which the analyte is first immobilized, directly or indirectly, upon a solid substrate.

3. A method as claimed in claim 1 or claim 2 in which domain A of said probe is a polypeptide capable of functioning as an antibody, and the probe is bound directly or indirectly to the analyte through domain A.

4. A method as claimed in any one of claims 1 to 3 in which the second domain B of said probe provides a promoter for DNA-dependent RNA polymerase activity derived from the bacteriophage T7.

5. A method as claimed in any one of claims 1 to 4 in which the second domain B of said probe is at least 10 and no more than 40 nucleotides long.

6. A method as claimed in any one of claims 1 to 5 in which the third domain C of said probe is at least 30 and no more than 80 nucleotides long.

7. A method as claimed in claim 4 wherein the polymerase employed is derived from the bacteriophage T7 and the sequence for the second domain B of said probe comprises the sequence:

5′-TAA TAC GAC TCA CTA TA-3′

3'-ATT ATG CTG AGT GAT AT-5'

8. A method as claimed in claim 4 wherein the polymerase employed is derived from the bacteriophage T7 and the nucleotide sequence of the second domain B of said probe is:

5′-CTG GCT TAT CGA AAT TAA TAC GAC TCA CTA TA-3′

3'-GAC CGA ATA GCT TTA ATT ATG CTG AGT GAT AT-5'

9. A method as claimed in any one of claims 1 to 8 which employs a probe in which the 5' residue of the upper strand of the nucleotide sequence for the third domain C, is adjacent to the second domain B, and is a guanosine residue.

10. A method as claimed in any one of claims 1 to 9 in which the sequence for the third domain C of said probe is:

5′-GGG AGA TGT GGT TGT CGT ACT TAG CGA AAT ACT GTC CGA GTC
G-3′

3'-CCC TCT ACA CCA ACA GCA TGA ATC GCT TTA TGA CAG GCT CAG
C-5'

11. A method as claimed in claim 10 which employs a probe in which the 3' end of the upper strand of the DNA nucleotide sequence of the second domain B is attached to the 5' end of the upper strand of the nucleotide sequence of domain C.

12. A method as claimed in any one of claims 1 to 11 in which a functional unit comprising the second and third domains, B and C, is present in said probe in multiple repeating units.

13. A method as claimed in any one of claims 1 to 12 in which:
(a) the third domain, C, of said probe is transcribed in the presence of labeled ribonucleotide triphosphates;
(b) the transcripts of the third domain have a first subdomain, c₁, complementary to an oligonucleotide capture probe which is immobilized on a solid substrate; and
(c) said transcripts are immobilized and quantified.

14. A method as claimed in any one of claims 1 to 12 in which:
(a) the third domain, C, of said probe is transcribed in the presence of biotinylated ribonucleotide triphosphates;
(b) the transcript of the third domain has a first subdomain, c₂, which is complementary to a labeled oligonucleotide probe;
(c) the transcripts are immobilized upon an avidinylated solid substrate; and
(d) the transcripts are quantified.

15. A method as claimed in any one of claims 1 to 12 in which:
(a) the third domain, C, of said probe is transcribed in the presence of both labeled and biotinylated ribonucleotide triphosphates;
(b) the transcripts are immobilized upon an avidinylated solid substrate; and
(c) the transcripts are quantified.

16. A method as claimed in any one of claims 1 to 12 in which:
(a) the transcript of the third domain, C, of the probe has two subdomains:
(i) a first subdomain, c₁, which is complementary to an oligonucleotide capture probe, the capture probe being immobilized on a solid substrate; and
(ii) a second subdomain, c₂, which is complementary to a labeled oligonucleotide probe;
(b) the transcript is hybridized to the capture probe;
(c) the labeled probe is hybridized to said transcript; and
(d) the labeled probe is quantified.

17. A method of constructing a probe as defined in any one of claims 1 and 3 to 12 which comprises combining domains A, B and C in covalent linkage.

18. A method of amplifying a detectable signal used to detect and quantify an antigen in an immunoassay which comprises:
(a) directly or indirectly binding to an analyte a biochemical molecular probe comprising a binding domain (A) which is a polypeptide covalently attached to a second domain (M) which comprises a multiplicity of single-stranded oligonucleotide subunits capable of hybridizing to a single-stranded labeled oligonucleotide, said multiplicity of single-stranded oligonucleotide subunits being covalently linked to each other;
(b) removing unbound probe;
(c) hybridizing single-stranded labeled oligonucleotides, which comprise a nucleotide sequence which is substantially complementary to the subunit sequence of the domain M probe, to the subunits of the second domain;
(d) removing unbound labeled oligonucleotide; and
(e) quantifying the amount of labeled oligonucleotide bound to the probe.

19. A method as claimed in claim 18 in which the analyte is first immobilized, directly or indirectly, on a solid substrate.

20. A method as claimed in claim 18 or claim 19 in which domain A is a polypeptide capable of functioning as an antibody, and the probe is bound directly or indirectly to the analyte via domain A.

21. A method as claimed in any one of claims 18 to 20 in which the polypeptide of domain A specifically binds to an epitope of an antibody which specifically binds to the analyte.

22. A method as claimed in any one of claims 18 to 21 employing a probe in which each oligonucleotide unit of the second domain, M, comprises about 15 to 50 bases and has a GC content in the range of about 40% to 60%.

23. A method as claimed in any one of claims 18 to 22 in which the second domain of said probe has a linear structure.

24. A method as claimed in any one of claims 18 to 22 in which the second domain of said probe has a branched structure.

25. A method as claimed in any one of claims 18 to 22 wherein at least a portion of the oligonucleotide subunits of the second domain of said probe are linked via a multifunctional moiety derived from a compound of the formula: 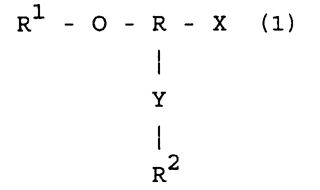 where R is an organic moiety, preferably a nucleic acid, R¹ is a hydroxyl protecting group that can be removed under conditions that do not remove synthetic nucleic acid from a solid phase and do not remove exocyclic nitrogen or phosphate protecting groups, X is a phosphorus-containing group that facilitates nucleic acid synthesis, Y is a radical derived from a nucleophilic group, and R² is R¹ or a blocking or protective group that can be removed and replaced with hydrogen without affecting R¹.

26. A method as claimed in any one of claims 18 to 22 wherein at least a portion of the oligonucleotide subunits of the second domain of said probe are linked via a multifunctional moiety derived from a compound of the formula: 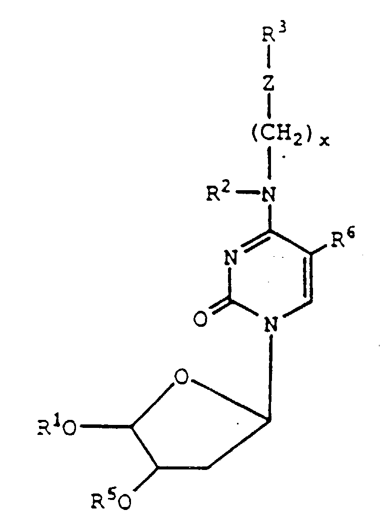 where Z is a nucleophile, R¹ is a protective group that is generally base-stable and acid sensitive, R² is hydrogen or methyl, R³ is a protective group that can be removed and replaced with hydrogen without affecting R¹, R⁵ is a phosphorus derivative that enables addition of nucleotides to the 5' position of an oligonucleotide chain during chemical synthesis, R⁶ is methyl, hydrogen, I, Br, or F, and X is an integer in the range of 1 to 8, inclusive.

27. A method as claimed in any one of claims 18 to 22 wherein at least a portion of the oligonucleotide subunits are linked via a multifunctional moiety derived from a compound of the formula: 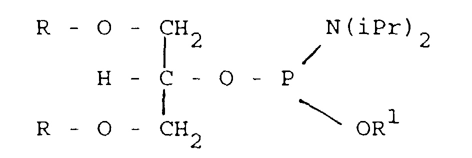 where R is said hydroxyl protecting group, iPr is isopropyl, and R¹ is methyl or beta-cyanoethyl.

28. A method of constructing a probe as defined in any one of claims 18 and 20 to 27 which comprises covalently linking domains A and M.

29. A method of amplifying a detectable signal in a competitive immunoassay which comprises:
(a) providing a molecular probe comprising:
(i) a first domain (A) which is capable of functioning as an immunogen;
(ii) a second domain (B) which is a double-stranded polynucleotide capable of functioning as a promoter for a DNA-dependent RNA polymerase enzyme activity; and
(iii) a third domain (C) which is either single- or double-stranded and adjacent to the second domain, such that the third domain is capable of functioning as a template for the promoter activity of the second domain;
(b) immobilizing the probe, directly or indirectly, upon a solid substrate in the presence of a competing analyte;
(c) removing unbound probe;
(d) transcribing multiple copies of RNA oligomers which are complementary to the template sequence, c', of the third domain, c, of the probe construct via a DNA-dependent RNA polymerase activity;
and
(e) quantifying the RNA transcripts.

30. A method of amplifying a detectable signal in a competitive immunoassay which comprises:
(a) providing a molecular probe comprising:
(i) a first domain (A) which is capable of functioning as an immunogen and is not an oligonucleotide; and
(ii) a second domain (M) comprising a multiplicity of single-stranded oligonucleotide subunits that are capable of binding specifically to a single-stranded nucleic acid sequence of interest.
(b) immobilizing the probe, directly or indirectly, upon a solid substrate in the presence of a competing analyte;
(c) removing unbound probe;
(d) hybridizing single-stranded labeled oligonucleotides, which comprise a nucleotide sequence which is substantially complementary to the subunit sequence of the domain M probe, to the subunits of the second domain;
(e) removing unbound labeled oligonucleotide; and
(f) quantifying the amount of labeled oligonucleotide bound to the probe.

31. A molecular probe for use as a signal amplifier in immunoassays comprising:
(a) a first domain (A) which is a polypeptide and functions as an antibody specific for a known antigen;
(b) a second domain (B) which is a double-stranded polynucleotide capable of functioning as a promoter for a DNA-dependent RNA polymerase enzyme activity; and
(c) a third domain (C) which is either single- or double-stranded and adjacent to the second domain, such that the third domain is capable of functioning as a template for the promoter activity of the second domain.

32. A molecular probe for use as a signal amplifier in immunoassays comprising:
(a) a first domain (A) comprising a polypeptide capable of functioning as an antibody which binds specifically to a known analyte; and
(b) a second domain (M) comprising a multiplicity of single-stranded oligonucleotide subunits that are capable of binding specifically to a single-stranded nucleic acid sequence of interest, said multiplicity of single-stranded oligonucleotide subunits being covalently linked to each other.

## Patentansprüche (Patentansprüche für folgende(n) Vertragsstaat(en): AT, BE, CH, LI, DE, DK, FR, GB, GR, IT, LU, NL, SE)

1. Molekulare Sonde zur Verwendung als Signalverstärker in Immunoassays, umfassend:
(a) Eine erste Domäne (A), welche ein Polypeptid ist und als ein Antikörper spezifisch für ein bekanntes Antigen fungiert;
(b) eine zweite Domäne (B), welche ein doppelsträngiges Polynucleotid ist, das fähig ist, als ein Promotor für eine DNA-abhängige RNA-Polymerase-Enzymaktivität zu fungieren; und
(c) eine dritte Domäne (C), welche entweder einzel- oder doppelaträngig ist und an die zweite Domäne derart angrenzt, daß die dritte Domäne fähig ist, als Matrize für die Promotoraktivität der zweiten Domäne zu fungieren.

2. Sonde nach Anspruch 1, dadurch gekennzeichnet, daß die zweite Domäne B einen Promotor für die DNA-abhängige RNA-Polymerase-Aktivität, abgeleitet vom Bakteriophagen T7, bereitstellt.

3. Sonde nach Anspruch 1 oder 2, dadurch gekennzeichnet, daß die zweite Domäne B mindestens 10 und nicht mehr als 40 Nucleotide lang ist.

4. Sonde nach einem der Ansprüche 1 bis 3, dadurch gekennzeichnet, daß die dritte Domäne C mindestens 30 und nicht mehr als 80 Nucleotide lang ist.

5. Sonde nach Anspruch 2, dadurch gekennzeichnet, daß die Sequenz der zweiten Domäne B die Sequenz 5'-TAA TAC GAC TCA CTA TA-3' 3'-ATT ATG CTG AGT GAT AT-5' umfaßt.

6. Sonde nach Anspruch 2, dadurch gekennzeichnet, daß die Nucleotidsequenz der zweiten Domäne B 5'-CTG GCT TAT CGA AAT TAA TAC GAC TCA CTA TA-3' 3'-GAC CGA ATA GCT TTA ATT ATG CTG AGT GAT AT-5' ist.

7. Sonde nach einem der Ansprüche 1 bis 6, dadurch gekennzeichnet, daß der 5'-Rest des oberen Strangs der Nucleotidsequenz der dritten Domäne C an die zweite Domäne B angrenzt und ein Guanosinrest ist.

8. Sonde nach einem der Ansprüche 1 bis 7, dadurch gekennzeichnet, daß die Sequenz der dritten Domäne C 
5'-GGG AGA TGT GGT TGT CGT ACT TAG CGA AAT ACT GTC CGA GTC
G-3'
 
3'-CCC TCT ACA CCA ACA GCA TGA ATC GCT TTA TGA CAG GCT CAG
C-5'
 ist.

9. Sonde nach Anspruch 8, dadurch gekennzeichnet, daß das 3'-Ende des oberen Strangs der DNA-Nucleotidsequenz der zweiten Domäne B am 5'-Ende des oberen Strangs der Nucleotidsequenz der Domäne C gebunden ist.

10. Sonde nach Anspruch 1, dadurch gekennzeichnet, daß das Transkript der dritten Domäne C zwei Unterdomänen besitzt:
(a) Eine erste Unterdomäne, c₁, welche fähig ist, an einen Oligonucleotid-Einfanglinker zu hybridisieren, wobei der Einfanglinker fähig ist, an ein Polynucleotid, welches auf einem Trägermaterial immobilisiert wurde, zu hybridisieren; und
(b) eine zweite Unterdomäne, c₂, welche fähig ist, an einen Oligonucleotid-Markierungslinker zu binden , wobei der Markierungslinker fähig ist, an eine quantifizierbare Sonde zu binden.

11. Sonde nach Anspruch 1, dadurch gekennzeichnet, daß eine funktionelle Einheit, umfassend die zweite und dritte Domäne B und C, in vielen, sich wiederholenden Einheiten vorhanden ist.

12. Verfahren zur Verstärkung eines nachweisbaren Signals in einem Immunoassay, welcher umfaßt:
(a) Direkte oder indirekte Bindung einer biochemischen molekularen Sonde, umfassend eine Bindungsdomäne A, welche ein Polypeptid ist, eine zweite Domäne B, welche eine doppelsträngige DNA-Sequenz ist, die fähig ist, als ein Promotor für eine DNA-abhängige RNA-Polymerase-Enzymaktivität zu fungieren, und eine dritte Domäne C, welche entweder einzel- oder doppelsträngig ist und an die zweite Domäne B angrenzt, derart, daß die dritte Domäne fähig ist, als Matrize für die Promotoraktivität der zweiten Domäne zu fungieren, an einen Analyten;
(b) Entfernung von nicht gebundener Sonde;
(c) Transkription vieler Kopien RNA-Oligomere, welche komplementär zu der Matrizensequenz c' der dritten Domäne C der konstruierten Sonde ist, mittels einer DNA-abhängigen RNA-Polymeraseaktivität; und
(d) Quantifizierung der RNA-Transkripte.

13. Verfahren nach Anspruch 12, dadurch gekennzeichnet, daß der Analyt zuerst direkt oder indirekt auf einem Trägermaterial immobilisiert wird.

14. Verfahren nach Anspruch 12 oder 13, dadurch gekennzeichnet, daß Domäne A ein Polypeptid ist, das fähig ist, als ein Antikörper zu fungieren, und die Sonde direkt oder indirekt über Domäne A an den Analyten gebunden wird.

15. Verfahren nach Anspruch 14, dadurch gekennzeichnet, daß eine Sonde gemäß einem der Ansprüche 2 bis 11 verwendet wird.

16. Verfahren nach einem der Ansprüche 12 bis 15, dadurch gekennzeichnet, daß die zweite Domäne B einen Promotor für die DNA-abhängige RNA-Polymerase des Bakteriophagen T7 bereitstellt.

17. Verfahren nach einem der Ansprüche 12 bis 16, dadurch gekennzeichnet, daß
(a) die dritte Domäne C der Sonde in Gegenwart markierter Ribonucleotid-Triphosphate transkribiert wird;
(b) die Transkripte der dritten Domäne eine erste Unterdomäne, c₁, komplementär zu einer Oligonucleotid-Einfangsonde, welche auf einem Trägermaterial immobilisiert ist, besitzen; und
(c) die Transkripte immobilisiert und quantifiziert werden.

18. Verfahren nach einem der Ansprüche 12 bis 16, dadurch gekennzeichnet, daß
(a) die dritte Domäne C der Sonde in Gegenwart biotinylierter Ribonucleotid-Triphosphate transkribiert wird;
(b) das Transkript der dritten Domäne eine erste Unterdomäne, c₂, besitzt, welche komplementär zu einer markierten Oligonucleotidsonde ist;
(c) die Transkripte auf einem avidinylierten Trägermaterial immobilisiert werden; und
(d) die Transkripte quantifiziert werden.

19. Verfahren nach einem der Ansprüche 12 bis 16, dadurch gekennzeichnet, daß
(a) die dritte Domäne C der Sonde in Gegenwart von sowohl markierten als auch biotinylierten Ribonucleotid-Triphosphaten transkribiert wird;
(b) die Transkripte auf einem avidinylierten Trägermaterial immobilisiert werden; und
(c) die Transkripte quantifiziert werden.

20. Verfahren nach einem der Ansprüche 12 bis 16, dadurch gekennzeichnet, daß
(a) das Transkript der dritten Domäne C der Sonde zwei Unterdomänen besitzt:
(i) eine erste Unterdomäne, c₁, welche komplementär zu einer Oligonucleotid-Einfangsonde ist, wobei die Einfangsonde auf einem Trägermaterial immobilisiert ist; und
(ii) eine zweite Unterdomäne, c₂, welche komplementär zu einer markierten Oligonucleotidsonde ist;
(b) das Transkript an die Einfangsonde hybridisiert wird;
(c) die markierte Sonde an das besagte Transkript hybridisiert wird; und
(d) die markierte Sonde quantifiziert wird.

21. Verfahren nach einem der Ansprüche 12 bis 20, dadurch gekennzeichnet, daß eine Sonde verwendet wird, in der eine Einheit, umfassend die zweite und dritte Domäne B und C, in vielen, sich wiederholenden Einheiten vorhanden ist.

22. Eine molekulare Sonde zur Verwendung als ein Signalverstärker in Immunoassays, umfassend:
(a) Eine erste Domäne (A), umfassend ein Polypeptid, das fähig ist, als ein Antikörper, welcher spezifisch an einen bekannten Analyten bindet, zu fungieren; und
(b) eine zweite Domäne (M), umfassend eine Vielzahl einzelsträngiger Oligonucleotid-Untereinheiten, die fähig sind, spezifisch an eine einzelsträngige Nucleinsäuresequenz von Interesse zu binden, wobei die Vielzahl der einzelsträngigen Oligonucleotid-Untereinheiten untereinander kovalent verbunden sind.

23. Sonde nach Anspruch 22, dadurch gekennzeichnet, daß jede Oligonucleotideinheit der zweiten Domäne M etwa 15 bis 50 Basen umfaßt und einen GC-Gehalt im Bereich von etwa 40% bis 60% besitzt.

24. Sonde nach Anspruch 22 oder 23, dadurch gekennzeichnet, daß die zweite Domäne eine lineare Struktur besitzt.

25. Sonde nach Anspruch 22 oder 23, dadurch gekennzeichnet, daß die zweite Domäne eine verzweigte Struktur besitzt.

26. Sonde nach Anspruch 22 oder 23, dadurch gekennzeichnet, daß zumindest ein Teil der oligonucleotid-Untereinheiten der zweiten Domäne über eine multifunktionelle Komponente, abgeleitet von einer Verbindung der Formel 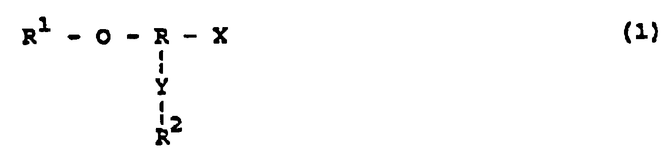 verbunden ist, wobei R ein organischer Rest ist, vorzugsweise eine Nucleinsäure, R¹ eine Hydroxyl-Schutzgruppe ist, welche unter Bedingungen entfernt werden kann, die eine synthetische Nucleinsäure nicht von einer Festphase entfernen, und exocyclische Stickstoff- oder Phosphat-Schutzgruppen nicht entfernen, X eine Phosphor-enthaltende Gruppe ist, welche die Nucleinsäuresynthese erleichtert, Y ein Rest, abgeleitet von einer nucleophilen Gruppe, ist und R² R¹ oder eine Blockierungs- oder Schutzgruppe ist, die entfernt und durch Wasserstoff ersetzt werden kann, ohne R¹ anzugreifen.

27. Sonde nach Anspruch 22 oder 23, dadurch gekennzeichnet, daß zumindest ein Teil der Oligonucleotid-Untereinheiten über eine multifunktionelle Komponente, abgeleitet von einer Verbindung der Formel 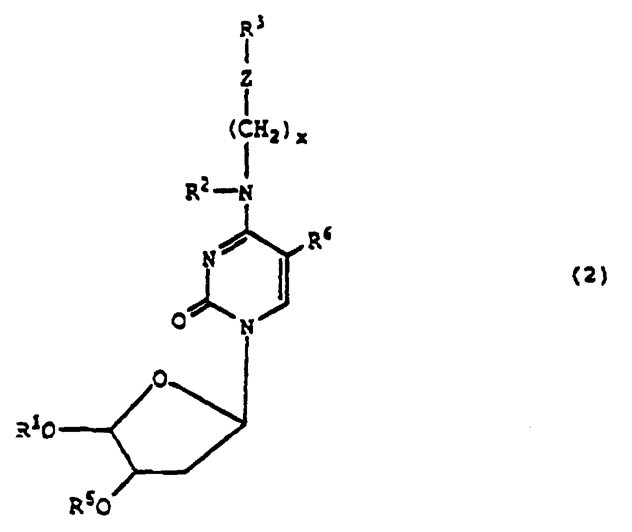 verbunden ist, wobei Z ein Nucleophil ist, R¹ eine Schutzgruppe ist, welche üblicherweise basenstabil und säureempfindlich ist, R² Wasserstoff oder Methyl ist, R³ eine Schutzgruppe ist, die entfernt und durch Wasserstoff ersetzt werden kann, ohne R¹ anzugreifen, R⁵ ein Phosphorderivat ist, welches die Addition von Nucleotiden an die 5'-Position einer Oligonucleotidkette während der chemischen Synthese ermöglicht, R⁶ Methyl, Wasserstoff, I, Br oder F ist und X eine ganze Zahl im Bereich von 1 bis 8 einschließlich ist.

28. Sonde nach Anspruch 22 oder 23, dadurch gekennzeichnet, daß zumindest ein Teil der Oligonucleotid-Untereinheiten über eine multifunktionelle Komponente, abgeleitet von einer Verbindung der Formel 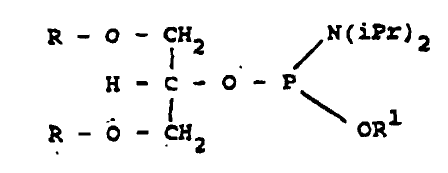 verbunden ist, wobei R die besagte Hydroxylschutzgruppe ist, iPr Isopropyl ist, und R¹ Methyl oder Beta-Cyanoethyl ist.

29. Verfahren zur Verstärkung eines nachweisbaren Signals, welches verwendet wird, um ein Antigen in einem Immunoassay nachzuweisen und zu quantifizieren, umfassend:
(a) Direkte oder indirekte Bindung einer biochemischen molekularen Sonde, umfassend eine Bindungsdomäne (A), welche ein Polypeptid ist, kovalent verbunden mit einer zweiten Domäne (M), welche eine Vielzahl einzelsträngiger oligomerer DNA-Untereinheiten umfaßt, die fähig sind, mit einem einzelsträngigen markierten Oligonucleotid zu hybridisieren, wobei die Vielzahl der einzelsträngigen Oligonucleotid-Untereinheiten untereinander kovalent verbunden sind, an einen Analyten;
(b) Entfernung nicht gebundener Sonde;
(c) Hybridisierung einzelsträngiger markierter Oligonucleotide, welche eine Nucleotidsequenz umfassen, welche im wesentlichen komplementär zu der Untereinheitensequenz der Sondendomäne M ist, an die Untereinheiten der zweiten Domäne;
(d) Entfernung von nicht gebundenem markiertem Oligonucleotid; und
(e) Quantifizierung der Menge an markiertem Oligonucleotid, welches an die Sonde gebunden ist.

30. Verfahren nach Anspruch 29, dadurch gekennzeichnet, daß der Analyt zuerst direkt oder indirekt auf einem Trägermaterial immobilisiert wird.

31. Verfahren nach Anspruch 29 oder 30, dadurch gekennzeichnet, daß Domäne A ein Polypeptid ist, das fähig ist, als ein Antikörper zu fungieren, und die Sonde direkt oder indirekt über Domäne A an den Analyten gebunden wird.

32. Verfahren nach einem der Ansprüche 29 bis 31, dadurch gekennzeichnet, daß das Polypeptid der Domäne A spezifisch an ein Epitop eines Antikörpers bindet, welcher spezifisch an den Analyten bindet.

33. Verfahren nach Anspruch 31, dadurch gekennzeichnet, daß eine Sonde gemäß einem der Ansprüche 23 bis 28 verwendet wird.

34. Verfahren zur Verstärkung eines nachweisbaren Signals in einem kompetitiven Immunoassay, umfassend:
(a) Die Bereitstellung einer molekularen Sonde, umfassend:
(i) Eine erste Domäne (A), welche fähig ist, als ein Immunogen zu fungieren;
(ii) eine zweite Domäne (B), welche ein doppelsträngiges Polynucleotid ist, das fähig ist, als ein Promotor für eine DNA-abhängige RNA-Polymerase- Enzymaktivität zu fungieren; und
(iii)eine dritte Domäne (C), welche entweder einzel- oder doppelsträngig ist und an die zweite Domäne derart angrenzt, daß die dritte Domäne fähig ist, als eine Matrize für die Promotor-Aktivität der zweiten Domäne zu fungieren;
(b) direkte oder indirekte Immobilisierung der Sonde auf einem Trägermaterial in Gegenwart eines konkurrierenden Analyten;
(c) Entfernung nicht gebundener Sonde;
(d) Transkription vieler Kopien RNA-Oligomere, welche komplementär zu der Matrizensequenz, c', der dritten Domäne, C, der konstruierten Sonde sind, mittels einer DNA-abhängigen RNA-Polymerase-Aktivität; und
(e) Quantifizierung der RNA-Transkripte.

35. Verfahren zur Verstärkung eines nachweisbaren Signals in einem kompetitiven Immunoassay, umfassend:
(a) Die Bereitstellung einer molekularen Sonde, umfassend:
(i) Eine erste Domäne (A), welche fähig ist, als ein Immunogen zu fungieren und kein Oligonucleotid ist; und
(ii) eine zweite Domäne (M), umfassend eine Vielzahl einzelsträngiger Oligonucleotid-Untereinheiten, welche fähig sind, spezifisch an eine einzelsträngige Nucleinsäuresequenz von Interesse zu binden;
(b) direkte oder indirekte Immobilisierung der Sonde auf einem Trägermaterial in Gegenwart eines konkurrierenden Analyten;
(c) Entfernung nicht gebundener Sonde;
(d) Hybridisierung einzelsträngiger markierter Oligonucleotide, welche eine Nucleotidsequenz umfassen, welche im wesentlichen komplementär zu der Untereinheitensequenz der Sondendomäne M ist, an die Untereinheiten der zweiten Domäne;
(e) Entfernung von nicht gebundenem markiertem Oligonucleotid; und
(f) Quantifizierung der Menge an markiertem Oligonucleotid, welches an die Sonde gebunden ist.

## Patentansprüche (Patentansprüche für folgende(n) Vertragsstaat(en): ES)

1. Verfahren zur Verstärkung eines nachweisbaren Signals in einem Immunoassay, umfassend:
(a) Direkte oder indirekte Bindung einer biochemischen molekularen Sonde, umfassend eine Bindungsdomäne A, welche ein Polypeptid ist, eine zweite Domäne B, welche eine doppelsträngige DNA-Sequenz ist, die fähig ist, als ein Promotor für eine DNA-abhängige RNA-Polymerase-Enzymaktivität zu fungieren, und eine dritte Domäne C, welche entweder einzel- oder doppelsträngig ist und an die zweite Domäne B angrenzt, derart, daß die dritte Domäne fähig ist, als Matrize für die Promotoraktivität der zweiten Domäne zu fungieren, an einen Analyten;
(b) Entfernung von nicht gebundener Sonde;
(c) Transkription vieler Kopien RNA-Oligomere, welche komplementär zu der Matrizensequenz c' der dritten Domäne C der konstruierten Sonde ist, mittels einer DNA-abhängigen RNA-Polymeraseaktivität; und
(d) Quantifizierung der RNA-Transkripte.

2. Verfahren nach Anspruch 1, dadurch gekennzeichnet, daß der Analyt zuerst direkt oder indirekt auf einem Trägermaterial immobilisiert wird.

3. Verfahren nach Anspruch 1 oder 2, dadurch gekennzeichnet, daß Domäne A der Sonde ein Polypeptid ist, das fähig ist, als ein Antikörper zu fungieren, und die Sonde direkt oder indirekt über Domäne A an den Analyten gebunden wird.

4. Verfahren nach einem der Ansprüche 1 bis 3, dadurch gekennzeichnet, daß die zweite Domäne B der Sonde einen Promotor für die DNA-abhängige RNA-Polymerase-Aktivität, abgeleitet aus dem Bakteriophagen T7, bereitstellt.

5. Verfahren nach einem der Ansprüche 1 bis 4, dadurch gekennzeichnet, daß die zweite Domäne B der Sonde mindestens 10 und nicht mehr als 40 Nucleotide lang ist.

6. Verfahren nach einem der Ansprüche 1 bis 5, dadurch gekennzeichnet, daß die dritte Domäne C der Sonde mindestens 30 und nicht mehr als 80 Nucleotide lang ist.

7. Verfahren nach Anspruch 4, dadurch gekennzeichnet, daß die verwendete Polymerase aus dem Bakteriophagen T7 abgeleitet ist und die Sequenz der zweiten Domäne B der Sonde die Sequenz 5'-TAA TAC GAC TCA CTA TA-3' 3'-ATT ATG CTG AGT GAT AT-5' umfaßt.

8. Verfahren nach Anspruch 4, dadurch gekennzeichnet, daß die verwendete Polymerase aus dem Bakteriophagen T7 abgeleitet ist und die Nucleotidsequenz der zweiten Domäne der Sonde B 5'-CTG GCT TAT CGA AAT TAA TAC GAC TCA CTA TA-3' 3'-GAC CGA ATA GCT TTA ATT ATG CTG AGT GAT AT-5' ist.

9. Verfahren nach einem der Ansprüche 1 bis 8, dadurch gekennzeichnet, daß eine Sonde verwendet wird, bei der der 5'-Rest des oberen Strangs der Nucleotidsequenz der dritten Domäne C an die zweite Domäne B angrenzt und ein Guanosinrest ist.

10. Verfahren nach einem der Ansprüche 1 bis 9, dadurch gekennzeichnet, daß die Sequenz der dritten Domäne C der Sonde 
5'-GGG AGA TGT GGT TGT CGT ACT TAG CGA AAT ACT GTC CGA GTC
G-3'
 
3'-CCC TCT ACA CCA ACA GCA TGA ATC GCT TTA TGA CAG GCT CAG
C-5'
 ist.

11. Verfahren nach Anspruch 10, dadurch gekennzeichnet, daß eine Sonde eingesetzt wird, bei der das 3'-Ende des oberen Strangs der DNA-Nucleotidsequenz der zweiten Domäne B am 5'-Ende des oberen Strangs der Nucleotidsequenz der Domäne C gebunden ist.

12. Verfahren nach einem der Ansprüche 1 bis 11, dadurch gekennzeichnet, daß eine funktionelle Einheit, umfassend die zweite und dritte Domäne B und C in der Sonde in vielen, sich wiederholenden Einheiten vorhanden ist.

13. Verfahren nach einem der Ansprüche 1 bis 12, dadurch gekennzeichnet, daß
(a) die dritte Domäne C der Sonde in Gegenwart markierter Ribonucleotid-Triphosphate transkribiert wird;
(b) die Transkripte der dritten Domäne eine erste Unterdomäne, c₁, komplementär zu einer Oligonucleotid-Einfangsonde, welche auf einem Trägermaterial immobilisiert ist, besitzen; und
(c) die Transkripte immobilisiert und quantifiziert werden.

14. Verfahren nach einem der Ansprüche 1 bis 12, dadurch gekennzeichnet, daß
(a) die dritte Domäne C der Sonde in Gegenwart biotinylierter Ribonucleotid-Triphosphate transkribiert wird;
(b) das Transkript der dritten Domäne eine erste Unterdomäne, c₂, besitzt, welche komplementär zu einer markierten Oligonucleotidsonde ist;
(c) die Transkripte auf einem avidinylierten Trägermaterial immobilisiert werden; und
(d) die Transkripte quantifiziert werden.

15. Verfahren nach einem der Ansprüche 1 bis 12, dadurch gekennzeichnet, daß
(a) die dritte Domäne C der Sonde in Gegenwart von sowohl markierten als auch biotinylierten Ribonucleotid-Triphosphaten transkribiert wird;
(b) die Transkripte auf einem avidinylierten Trägermaterial immobilisiert werden; und
(c) die Transkripte quantifiziert werden.

16. Verfahren nach einem der Ansprüche 1 bis 12, dadurch gekennzeichnet, daß
(a) das Transkript der dritten Domäne C der Sonde zwei Unterdomänen besitzt:
(i) eine erste Unterdomäne, c₁, welche komplementär zu einer Oligonucleotid-Einfangsonde ist, wobei die Einfangsonde auf einem Trägermaterial immobilisiert ist; und
(ii) eine zweite Unterdomäne, c₂, welche komplementär zu einer markierten Oligonucleotidsonde ist;
(b) das Transkript an die Einfangsonde hybridisiert wird;
(c) die markierte Sonde an das besagte Transkript hybridisiert wird; und
(d) die markierte Sonde quantifiziert wird.

17. Verfahren zur Konstruktion einer Sonde, wie in einem der Ansprüche 1 und 3 bis 12 definiert, dadurch gekennzeichnet, daß es die Kombination der Domänen A, B und C in kovalenter Bindung umfaßt.

18. Verfahren zur Verstärkung eines nachweisbaren Signals, welches verwendet wird, um ein Antigen in einem Immunoassay nachzuweisen und zu quantifizieren, umfassend:
(a) Direkte oder indirekte Bindung einer biochemischen molekularen Sonde, umfassend eine Bindungsdomäne (A), welche ein Polypeptid ist, kovalent verbunden mit einer zweiten Domäne (M), welche eine Vielzahl einzelsträngiger Oligonucleotid-Untereinheiten umfaßt, die fähig sind, mit einem einzelsträngigen markierten Oligonucleotid zu hybridisieren, wobei die Vielzahl der einzelsträngigen Oligonucleotid-Untereinheiten untereinander kovalent verbunden sind, an einen Analyten;
(b) Entfernung nicht gebundener Sonde;
(c) Hybridisierung einzelsträngiger markierter Oligonucleotide, welche eine Nucleotidsequenz umfassen, welche im wesentlichen komplementär zu der Untereinheitensequenz der Sondendomäne M ist, an die Untereinheiten der zweiten Domäne;
(d) Entfernung von nicht gebundenem markiertem Oligonucleotid; und
(e) Quantifizierung der Menge an markiertem Oligonucleotid, welches an die sonde gebunden ist.

19. Verfahren nach Anspruch 18, dadurch gekennzeichnet, daß der Analyt zuerst direkt oder indirekt auf einem Trägermaterial immobilisiert wird.

20. Verfahren nach Anspruch 18 oder 19, dadurch gekennzeichnet, daß Domäne A ein Polypeptid ist, das fähig ist, als ein Antikörper zu fungieren, und die Sonde direkt oder indirekt über Domäne A an den Analyten gebunden wird.

21. Verfahren nach einem der Ansprüche 18 bis 20, dadurch gekennzeichnet, daß das Polypeptid der Domäne A spezifisch an ein Epitop eines Antikörpers bindet, welcher spezifisch an den Analyten bindet.

22. Verfahren nach einem der Ansprüche 18 bis 21, dadurch gekennzeichnet, daß eine Sonde verwendet wird, in der jede Oligonucleotideinheit der zweiten Domäne M etwa 15 bis 50 Basen umfaßt und einen GC-Gehalt im Bereich von etwa 40% bis 60% besitzt.

23. Verfahren nach einem der Ansprüche 18 bis 22, dadurch gekennzeichnet, daß die zweite Domäne der Sonde eine lineare Struktur besitzt.

24. Verfahren nach einem der Ansprüche 18 bis 22, dadurch gekennzeichnet, daß die zweite Domäne der Sonde eine verzweigte Struktur besitzt.

25. Verfahren nach einem der Ansprüche 18 bis 22, dadurch gekennzeichnet, daß zumindest ein Teil der Oligonucleotid-Untereinheiten der zweiten Domäne der Sonde über eine multifunktionelle Komponente, abgeleitet von einer Verbindung der Formel 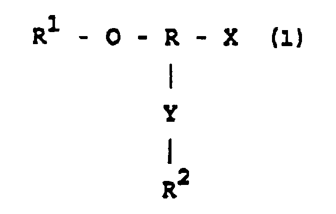 verbunden ist, wobei R ein organischer Rest ist, vorzugsweise eine Nucleinsäure, R¹ eine Hydroxyl-Schutzgruppe ist, welche unter Bedingungen entfernt werden kann, die eine synthetische Nucleinsäure nicht von einer Festphase entfernen, und exocyclische Stickstoff- oder Phosphat-Schutzgruppen nicht entfernen, X eine Phosphor-enthaltende Gruppe ist, welche die Nucleinsäuresynthese erleichtert, Y ein Rest, abgeleitet von einer nucleophilen Gruppe, ist und R² R¹ oder eine Blockierungs- oder Schutzgruppe ist, die entfernt und durch Wasserstoff ersetzt werden kann, ohne R¹ anzugreifen.

26. Verfahren nach einem der Ansprüche 18 bis 22, dadurch gekennzeichnet, daß zumindest ein Teil der Oligonucleotid-Untereinheiten der zweiten Domäne der Sonde über eine multifunktionelle Komponente, abgeleitet von einer Verbindung der Formel 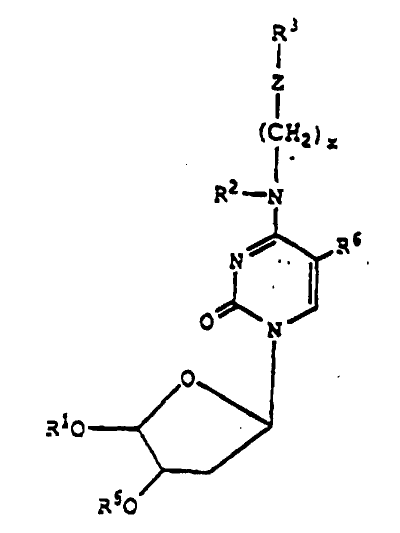 verbunden ist, wobei Z ein Nucleophil ist, R¹ eine Schutzgruppe ist, welche üblicherweise basenstabil und säureempfindlich ist, R² Wasserstoff oder Methyl ist, R³ eine Schutzgruppe ist, die entfernt und durch Wasserstoff ersetzt werden kann, ohne R¹ anzugreifen, R⁵ ein Phosphorderivat ist, welches die Addition von Nucleotiden an die 5'-Position einer Oligonucleotidkette während der chemischen Synthese ermöglicht, R⁶ Methyl, Wasserstoff, I, Br oder F ist und X eine ganze Zahl im Bereich von 1 bis 8 einschließlich ist.

27. Verfahren nach einem der Ansprüche 18 bis 22, dadurch gekennzeichnet, daß zumindest ein Teil der Oligonucleotid-Untereinheiten über eine multifunktionelle Komponente, abgeleitet von einer Verbindung der Formel 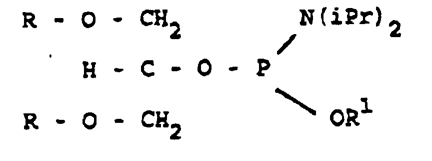 verbunden ist, wobei R die besagte Hydroxyl-Schutzgruppe ist, iPr Isopropyl ist, und R¹ Methyl oder Beta-Cyanoethyl ist.

28. Verfahren zur Konstruktion einer Sonde, wie in einem der Ansprüche 18 und 20 bis 27 definiert, dadurch gekennzeichnet, daß es das kovalente Verbinden der Domänen A und M umfaßt.

29. Verfahren zur Verstärkung eines nachweisbaren Signals in einem kompetitiven Immunoassay, umfassend:
(a) Die Bereitstellung einer molekularen Sonde, umfassend:
(i) Eine erste Domäne (A), welche fähig ist, als ein Immunogen zu fungieren;
(ii) eine zweite Domäne (B), welche ein doppelsträngiges Polynucleotid ist, das fähig ist, als ein Promotor für eine DNA-abhängige RNA-Polymerase-Enzymaktivität zu fungieren; und
(iii)eine dritte Domäne (C), welche entweder einzel- oder doppelsträngig ist und an die zweite Domäne derart angrenzt, daß die dritte Domäne fähig ist, als eine Matrize für die Promotor-Aktivität der zweiten Domäne zu fungieren;
(b) direkte oder indirekte Immobilisierung der Sonde auf einem Trägermaterial in Gegenwart eines konkurrierenden Analyten;
(c) Entfernung nicht gebundener Sonde;
(d) Transkription vieler Kopien RNA-Oligomere, welche komplementär zu der Matrizensequenz, c', der dritten Domäne, C, der konstruierten Sonde sind, mittels einer DNA-abhängigen RNA-Polymerase-Aktivität; und
(e) Quantifizierung der RNA-Transkripte.

30. Verfahren zur Verstärkung eines nachweisbaren Signals in einem kompetitiven Immunoassay, umfassend:
(a) Die Bereitstellung einer molekularen Sonde, umfassend:
(i) Eine erste Domäne (A), welche fähig ist, als ein Immunogen zu fungieren und kein Oligonucleotid ist; und
(ii) eine zweite Domäne (M), umfassend eine Vielzahl einzelsträngiger Oligonucleotid-Untereinheiten, welche fähig sind, spezifisch an eine einzelsträngige Nucleinsäuresequenz von Interesse zu binden;
(b) direkte oder indirekte Immobilisierung der Sonde auf einem Trägermaterial in Gegenwart eines konkurrierenden Analyten;
(c) Entfernung nicht gebundener Sonde;
(d) Hybridisierung einzelsträngiger markierter Oligonucleotide, welche eine Nucleotidsequenz umfassen, welche im wesentlichen komplementär zu der Untereinheitensequenz der Sondendomäne M ist, an die Untereinheiten der zweiten Domäne;
(e) Entfernung von nicht gebundenem markiertem Oligonucleotid; und
(f) Quantifizierung der Menge an markiertem Oligonucleotid, welches an die Sonde gebunden ist.

31. Eine molekulare Sonde zur Verwendung als Signalverstärker in Immunoassays, umfassend:
(a) Eine erste Domäne (A), welche ein Polypeptid ist und als ein Antikörper spezifisch für ein bekanntes Antigen fungiert;
(b) eine zweite Domäne (B), welche ein doppelsträngiges Polynucleotid ist, das fähig ist, als ein Promotor für eine DNA-abhängige RNA-Polymerase-Enzymaktivität zu fungieren; und
(c) eine dritte Domäne (C), welche entweder einzel- oder doppelsträngig ist und an die zweite Domäne derart angrenzt, daß die dritte Domäne fähig ist, als Matrize für die Promotoraktivität der zweiten Domäne zu fungieren.

32. Eine molekulare Sonde zur Verwendung als ein Signalverstärker in Immunoassays, umfassend:
(a) Eine erste Domäne (A), umfassend ein Polypeptid, das fähig ist, als ein Antikörper, welcher spezifisch an einen bekannten Analyten bindet, zu fungieren; und
(b) eine zweite Domäne (M), umfassend eine Vielzahl einzelsträngiger Oligonucleotid-Untereinheiten, die fähig sind, spezifisch an eine einzelsträngige Nucleinsäuresequenz von Interesse zu binden, wobei die Vielzahl der einzelsträngigen Oligonucleotid-Untereinheiten untereinander kovalent verbunden sind.

## Revendications (Revendications pour l'(les) Etat(s) contractant(s) suivant(s): AT, BE, CH, LI, DE, DK, FR, GB, GR, IT, LU, NL, SE)

1. Une sonde moléculaire pour utilisation comme amplificateur de signal dans des tests immunologiques comprenant :
(a) un premier domaine (A) qui est un polypeptide et fonctionne comme un anticorps spécifique pour un antigène connu ;
(b) un second domaine (B) qui est un polynucléotide double brin capable de fonctionner comme un promoteur pour une activité enzymatique d'ARN polymérase ADN dépendante ; et
(c) un troisième domaine (C) qui est soit simple soit double brin et adjacent au second domaine, de façon que le troisième domaine soit capable de fonctionner comme une matrice pour l'activité du promoteur du second domaine.

2. Une sonde telle que revendiquée à la revendication 1 dans laquelle ledit second domaine B fournit un promoteur pour l'activité d'ARN polymérase ADN dépendante dérivée du bactériophage T7.

3. Une sonde telle que revendiquée à la revendication 1 ou 2 dans laquelle le second domaine B a une longueur d'au moins 10 et pas plus de 40 nucléotides.

4. Une sonde telle que revendiquée dans l'une quelconque des revendications 1 à 3 dans laquelle le troisième domaine C a une longueur d'au moins 30 et pas plus de 80 nucléotides.

5. Une sonde telle que revendiquée à la revendication 2 dans laquelle la séquence du second domaine B comprend la séquence :

5'-TAA TAC GAC TCA CTA TA-3'

3'-ATT ATG CTG AGT GAT AT-5'

6. Une sonde telle que revendiquée à la revendication 2 dans laquelle la séquence nucléotidique du second domaine B est :

5'-CTG GCT TAT CGA AAT TAA TAC GAC TCA CTA TA-3'

3'-GAC CGA ATA GCT TTA ATT ATG CTG AGT GAT AT-5'

7. Une sonde telle que revendiquée dans l'une quelconque des revendications 1 à 6 dans laquelle le résidu en 5' du brin supérieur de la séquence nucléotidique du troisième domaine C est adjacent au second domaine B et est un résidu guanosine.

8. Une sonde telle que revendiquée dans l'une quelconque des revendications 1 à 7 dans laquelle la séquence du troisième domaine C est :

5'-GGG AGA TGT GGT TGT CGT ACT TAG CGA AAT ACT GTC CGA GTC
G-3'

3′-CCC TCT ACA CCA ACA GCA TGA ATC GCT TTA TGA CAG GCT CAG
C-5′

9. Une sonde telle que revendiquée à la revendication 8 dans laquelle l'extrémité 3' du brin supérieur de la séquence nucléotidique d'ADN du second domaine B est attachée à l'extrémité 5' du brin supérieur de la séquence nucléotidique du domaine C.

10. Une sonde telle que revendiquée à la revendication 1 dans laquelle le transcrit du troisième domaine C a deux sous-domaines :
(a) un premier sous-domaine, c₁, qui est capable de s'hybrider avec un linker de capture oligonucléotidique, le linker de capture étant capable de s'hybrider avec un polynucléotide immobilisé sur un substrat solide ; et
(b) un second sous-domaine, c₂, qui est capable de se lier à un linker marqueur oligonucléotidique, le linker marqueur étant capable de se lier à une sonde quantifiable.

11. Une sonde telle que revendiquée à la revendication 1 dans laquelle une unité fonctionnelle comprenant les second et troisième domaines, B et C, est présente en unités répétitives multiples.

12. Une méthode d'amplification d'un signal détectable dans un test immunologique qui comprend :
(a) la liaison directe ou indirecte à un analyte d'une sonde moléculaire biochimique comprenant un domaine de liaison (A) qui est un polypeptide, un second domaine (B) qui est une séquence d'ADN double brin capable de fonctionner comme un promoteur pour une activité enzymatique d'ARN polymérase ADN dépendante, et un troisième domaine (C) qui est soit simple soit double brin et adjacent au second domaine B, de façon que le troisième domaine soit capable de fonctionner comme une matrice pour l'activité de promoteur du second domaine :
(b) l'élimination de la sonde non liée ;
(c) la transcription de copies multiples d'oligomères d'ARN qui sont complémentaires de la séquence matrice, c', du troisième domaine, C, de la construction servant de sonde via une activité d'ARN polymérase ADN dépendante ; et
(d) la quantification des transcrits d'ARN.

13. Une méthode talle que revendiquée à la revendication 12 dans laquelle l'analyte est d'abord immobilisé, directement ou indirectement, sur un substrat solide.

14. Une méthode telle que revendiquée à la revendication 12 ou le revendication 13 dans laquelle le domaine A est un polypeptide capable de fonctionner comme un anticorps, et la sonde est liée directement ou indirectement à l'analyte par l'intermédiaire du domaine A.

15. Une méthode telle que revendiquée à la revendication 14 qui utilise une sonde selon l'une quelconque des revendications 2 a 11.

16. Une méthode telle que revendiquée dans l'une quelconque des revendications 12 à 15 dans laquelle le second domaine, B, fournit un promoteur pour l'ARN polymérase ADN dépendante du bactériophage T7.

17. Une méthode telle que revendiquée dans l'une quelconque des revendications 12 à 16 dans laquelle :
(a) le troisième domaine, C, de ladite sonde est transcrit en présence de ribonucléotides triphosphates marqués ;
(b) les transcrits du troisième domaine ont un premier sous-domaine, c₁, complémentaire d'une sonde de capture oligonucléotidique qui est immobilisée sur un substrat solide ; et
(c) lesdits transcrits sont immobilisés et quantifiés.

18. Une méthode telle que revendiquée dans l'une quelconque des revendications 12 à 16 dans laquelle :
(a) le troisième domaine, C, de ladite sonde est transcrit en présence de ribonucléotides triphosphates biotinylés ;
(b) le transcrit du troisième domaine a un sous-domaine, c₂, qui est complémentaire d'une sonde oligonucléotidique marquée ;
(c) les transcrits sont immobilisés sur un substrat solide avidinylé ; et
(d) les transcrits sont quantifiés.

19. Une méthode telle que revendiquée dans l'une quelconque des revendications 12 à 16 dans laquelle :
(a) le troisième domaine, C, de la sonde est transcrit en présence à la fois de ribonucléotides triphosphates marqués et biotinylés :
(b) les transcrits sont immobilisés sur un substrat solide avidinylé ; et
(c) les transcrits sont quantifiés.

20. Une méthode telle que revendiquée dans l'une quelconque des revendications 12 à 16 dans laquelle :
(a) le transcrit du troisième domaine, C, de ladite sonde a deux sous-domaines :
(i) un premier sous-domaine, c₁, qui est complémentaire d'une sonde de capture oligonucléotidique, la sonde de capture étant immobilisée sur un substrat solide ; et
(il) un second sous-domaine, c₂, qui est complémentaire d'une sonde oligonucléotidique marquée ;
(b) le transcrit est hybridé avec la sonde de capture ;
(c) la sonde marquée est hybridée avec ledit transcrit ; et
(d) la sonde marquée est quantifiée.

21. Une méthode telle que revendiquée dans l'une quelconque des revendications 12 à 20 dans laquelle on utilise une sonde dans laquelle une unité comprenant les second et troisième domaines, B et C, est présente en unités répétitives multiples.

22. Une sonde moléculaire pour utilisation comme amplificateur de signal dans des tests immunologiques comprenant :
(a) un premier domaine (A) comprenant un polypeptide capable de fonctionner comme un anticorps qui se lie spécifiquement à un analyte connu ; et
(b) un second domaine (M) comprenant une multiplicité de sous-unités oligonucléotidiques simple brin qui sont capables de se lier spécifiquement à une séquence d'acide nucléique simple brin d'intérêt, ladite multiplicité de sous-unités oligonucléotidiques simple brin étant liées de manière covalente les unes aux autres.

23. Une sonde telle que revendiquée à la revendication 22 dans laquelle chaque unité oligonucléotidique du second domaine, M, comprend environ 15 à 50 bases et a un contenu en GC dans la gamme d'environ 40% à 60%.

24. Une sonde telle que revendiquée à la revendication 22 ou la revendication 23 dans laquelle le second domaine a une structure linéaire.

25. Une sonde telle que revendiquée la revendication 22 ou la revendication 23 dans laquelle le second domaine a une structure branchée.

26. Une sonde telle que revendiquée à la revendication 22 ou la revendication 23 dans laquelle au moins une portion des sous-unités oligonucléotidiques du second domaine est liée via une partie multifonctionnelle dérivée d'un composé de formule : 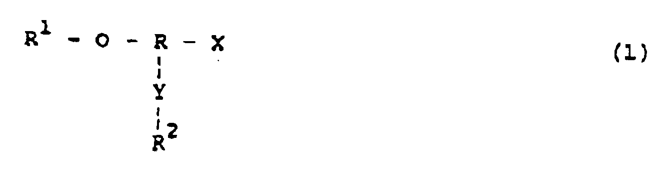 dans laquelle R est une partie organique, de préférence un acide nucléique, R¹ est un groupe protecteur d'hydroxyle qui peut être éliminé dans des conditions qui ne retirent pas l'acide nucléique de synthèse d'une phase solide et n'éliminent pas les groupes protecteurs d'azote exocyclique ou de phosphate, X est un groupe contenant du phosphore qui facilite la synthèse d'acide nucléique, Y est un radical dérivé d'un groupe nucléophile, et R² est R¹ ou un groupe bloquant ou protecteur qui peut être éliminé et remplacé par l'hydrogène sans affecter R¹.

27. Une sonde telle que revendiquée à la revendication 22 ou la revendication 23 dans laquelle au moins une portion des sous-unités oligonucléotidiques est liée via une partie multifonctionnelle dérivée d'un composé de formule : 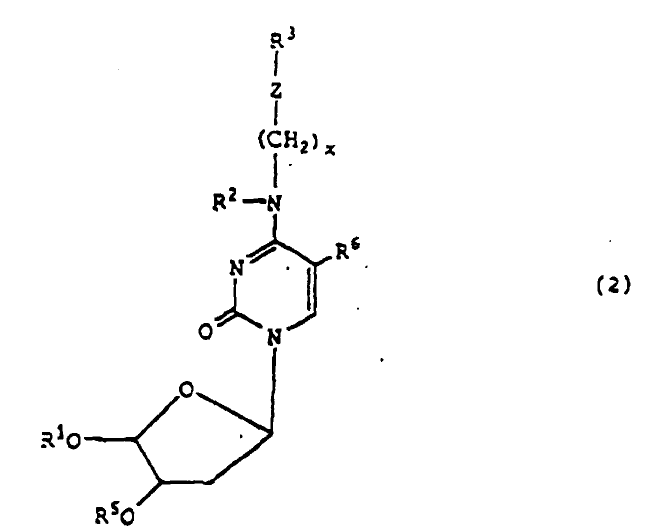 dans laquelle Z est un nucléophile, R¹ est un groupe protecteur qui est généralement résistant aux bases et sensible aux acides, R² est l'hydrogène ou un méthyle, R³ est un groupe protecteur qui peut être éliminé et remplacé par l'hydrogène sans affecter R¹, R⁵ est un dérivé du phosphore qui permet l'addition de nucléotides en position 5' d'une chaîne oligonucléotidique durant la synthèse chimique, R6 est un méthyle, un hydrogène, I, Br, ou F, et X est un nombre entier dans la gamme de 1 à 8, inclus.

28. Une sonde telle que revendiquée à la revendication 22 ou la revendication 23 dans laquelle au moins une portion des sous-unités oligonucléotidiques est liée via une partie multifonctionnelle dérivée d'un composé de formule : 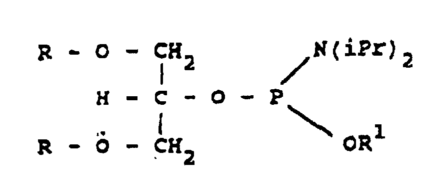 dans laquelle R est ledit groupe protecteur d'hydroxyle, iPr est un isopropyle, et R¹ est un méthyle ou un β-cyanoéthyle.

29. Une méthode d'amplification d'un signal détectable utilisée pour détecter et quantifier un antigène dans un test immunologique qui comprend :
(a) la liaison directe ou indirecte à un analyte d'une sonde moléculaire biochimique comprenant un domaine de liaison (A) qui est un polypeptide attaché de manière covalente à un second domaine (M) qui comprend une multiplicité de sous-unités oligomériques d'ADN simple brin capable de s'hybrider à un oligonucléotide marqué simple brin, ladite multiplicité de sous-unités oligonucléotidiques simple brin étant liées de manière covalente les unes aux autres ;
(b) l'élimination de la sonde non liée ;
(c) l'hybridation d'oligonucléotides marqués simple brin, qui comprennent une séquence nucléotidique qui est substantiellement complémentaire de la séquence de la sous-unité de la sonde du domaine M, avec les sous-unités du second domaine ;
(d) l'élimination de l'oligonucléotide marqué non lié ; et
(e) la quantification de la quantité d'oligonucléotide marqué lié à la sonde.

30. Une méthode telle que revendiquée à la revendication 29 dans laquelle l'analyte est d'abord immobilisé, directement ou indirectement, sur un substrat solide.

31. Une méthode telle que revendiquée à la revendication 29 ou la revendication 30 dans laquelle le domaine A est un polypeptide capable de fonctionner comme un anticorps, et la sonde est liée directement ou indirectement l'analyte via le domaine A.

32. Une méthode telle que revendiquée dans l'une quelconque des revendications 29 à 31 dans laquelle le polypeptide du domaine A se lie spécifiquement à un épitope d'un anticorps qui se lie spécifiquement a l'analyte.

33. Une méthode telle que revendiquée à la revendication 31 dans laquelle une sonde selon l'une quelconque des revendications 23 à 28 est utilisée.

34. Une méthode d'amplification d'un signal détectable dans un test immunologique de compétition qui comprend :
(a) la fourniture d'une sonde moléculaire comprenant :
(i) un premier domaine (A) qui est capable de fonctionner comme un immunogène ;
(il) un second domaine (B) qui est un polynucléotide double brin capable de fonctionner comme un promoteur pour une activité enzymatique d'ARN polymérase ADN dépendante ; et
(iii) un troisième domaine (C) qui est soit simple soit double brin et adjacent au second domaine, de façon que le troisième domaine soit capable de fonctionner comme une matrice pour l'activité de promoteur du second domaine ;
(b) l'immobilisation de la sonde, directement ou indirectement, sur un substrat solide en présence d'un analyte entrant en compétition ;
(c) l'élimination de la sonde non liée ;
(d) la transcription de copies multiples d'oligomères d'ARN qui sont complémentaires de la séquence matrice, c', du troisième domaine, c, de la construction servant de sonde via une activité d'ARN polymérase ADN dépendante ; et
(e) la quantification des transcrits d'ARN.

35. Une méthode d'amplification d'un signal détectable dans un test immunologique de compétition qui comprend :
(a) la fourniture d'une sonde moléculaire comprenant :
(i) un premier domaine (A) qui est capable de fonctionner comme un immunogène et n'est pas un oligonucléotide ; et
(ii) un second domaine (M) comprenant une multiplicité de sous-unités oligonucléotidiques simple brin qui sont capables de se lier spécifiquement à une séquence d'acide nucléique simple brin d'intérêt.
(b) l'immobilisation de la sonde, directement ou indirectement, sur un substrat solide en présence d'un analyte entrant en compétition ;
(c) l'élimination de la sonde non liée ;
(d) l'hybridation d'oligonucléotides marqués simple brin, qui comprennent une séquence nucléotidique qui est substantiellement complémentaire de la séquence de sous-unités de la sonde du domaine M, avec les sous-unités du second domaine ;
(e) l'élimination de l'oligonucléotide marqué non lié ; et
(f) la quantification de la quantité d'oligonucléotide marqué lié à la sonde.

## Revendications (Revendications pour l'(les) Etat(s) contractant(s) suivant(s): ES)

1. Une méthode d'amplification d'un signal détectable dans un test immunologique qui comprend :
(a) la liaison directe ou indirecte à un analyte d'une sonde moléculaire biochimique comprenant un domaine de liaison (A) qui est un polypeptide, un second domaine (B) qui est une séquence d'ADN double brin capable de fonctionner comme un promoteur pour une activité enzymatique d'ARN polymérase ADN dépendante, et un troisième domaine (C) qui est soit simple soit double brin et adjacent au second domaine B, de façon que le troisième domaine soit capable de fonctionner comme une matrice pour l'activité du promoteur du second domaine ;
(b) l'élimination de la sonde non liée ;
(c) la transcription de copies multiples d'oligomères d'ARN qui sont complémentaires de la séquence matrice, c', du troisième domaine, C, de la construction sonde via une activité d'ARN polymérase ADN dépendante ; et
(d) la quantification des transcrits d'ARN.

2. Une méthode telle que revendiquée à la revendication 1 dans laquelle l'analyte est d'abord immobilisé, directement ou indirectement, sur un substrat solide.

3. Une méthode telle que revendiquée à la revendication 1 ou 2 dans laquelle le domaine A de ladite sonde est un polypeptide capable de fonctionner comme un anticorps, et la sonde est liée directement ou indirectement à l'analyte par l'intermédiaire du domaine A.

4. Une méthode telle que revendiquée dans l'une quelconque des revendications 1 à 3 dans laquelle le second domaine B de ladite sonde fournit un promoteur pour l'activité d'ARN polymérase ADN dépendante dérivée du bactériophage T7.

5. Une méthode telle que revendiquée dans l'une quelconque des revendications 1 à 4 dans laquelle le second domaine B de ladite sonde a une longueur d'au moins 10 et pas plus de 40 nucléotides.

6. Une méthode telle que revendiquée dans l'une quelconque des revendications 1 à 5 dans laquelle le troisième domaine C de ladite sonde a une longueur d'au moins 30 et pas plus de 80 nucléotides.

7. Une méthode telle que revendiquée à la revendication 4 dans laquelle la polymérase utilisée est dérivée du bactériophage T7 et la séquence du second domaine B de ladite sonde comprend la séquence :

5'-TAA TAC GAC TCA CTA TA-3'

3'-ATT ATG CTG AGT GAT AT-5'

8. Une méthode telle que revendiquée à la revendication 4 dans laquelle la polymérase utilisée est dérivée du bactériophage T7 et la séquence nucléotidique du second domaine B de ladite sonde est :

5'-CTG GCT TAT CGA AAT TAA TAC GAC TCA CTA TA-3'

3'-GAC CGA ATA GCT TTA ATT ATG CTG AGT GAT AT-5'

9. Une méthode telle que revendiquée dans l'une quelconque des revendications 1 à 8 qui utilise une sonde dans laquelle le résidu en 5' du brin supérieur de la séquence nucléotidique du troisième domaine C est adjacent au second domaine B et est un résidu guanosine.

10. Une méthode telle que revendiquée dans l'une quelconque des revendications 1 à 9 dans laquelle la séquence du troisième domaine C de ladite sonde est :

5'-GGG AGA TGT GGT TGT CGT ACT TAG CGA AAT ACT GTC CGA GTC
G-3'

3′-CCC TCT ACA CCA ACA GCA TGA ATC GCT TTA TGA CAG GCT CAG
C-5′

11. Une méthode telle que revendiquée à la revendication 10 qui utilise une sonde dans laquelle l'extrémité 3' du brin supérieur de la séquence nucléotidique d'ADN du second domaine B est attachée à l'extrémité 5' du brin supérieur de la séquence nucléotidique du domaine C.

12. Une méthode telle que revendiquée dans l'une quelconque des revendications 1 à 11 dans laquelle une unité fonctionnelle comprenant les second et troisième domaines, B et C, est présente dans ladite sonde en unités répétitives multiples.

13. une méthode telle que revendiquée dans l'une quelconque des revendications 1 à 12 dans laquelle :
(a) le troisième domaine, C, de ladite sonde est transcrit en présence de ribonucléotides triphosphates marqués ;
(b) les transcrits du troisième domaine ont un premier sous-domaine, c₁, complémentaire d'une sonde de capture oligonucléotidique qui est immobilisée sur un substrat solide ; et
(c) lesdits transcrits sont immobilisés et quantifiés.

14. Une méthode telle que revendiquée dans l'une quelconque des revendications 1 à 12 dans laquelle :
(a) le troisième domaine, C, de ladite sonde est transcrit en présence de ribonucléotides triphosphates biotinylés ;
(b) le transcrit du troisième domaine a un premier sous-domaine, ci, qui est complémentaire d'une sonde oligonucléotidique marquée ;
(c) les transcrits sont immobilisés sur un substrat solide avidinylé ; et
(d) les transcrits sont quantifiés.

15. Une méthode telle que revendiquée dans l'une quelconque des revendications 1 à 12 dans laquelle :
(a) le troisième domaine, C, de ladite sonde est transcrit en présence à la fois de ribonucléotides triphosphates marqués et biotinylés ;
(b) les transcrits sont immobilisés sur un substrat solide avidinylé ; et
(c) les transcrits sont quantifiés.

16. Une méthode telle que revendiquée dans l'une quelconque des revendications 1 à 12 dans laquelle :
(a) le transcrit du troisième domaine, C, de la sonde a deux sous-domaines :
(i) un premier sous-domaine, c₁, qui est complémentaire d'une sonde de capture oligonucléotidique, la sonde de capture étant immobilisée sur un substrat solide ; et
(ii) un second sous-domaine, c₂, qui est complémentaire d'une sonde oligonucléotidique marquée ;
(b) le transcrit est hybridé avec la sonde de capture ;
(c) la sonde marquée est hybridée avec ledit transcrit ; et
(d) la sonde marquée est quantifiée.

17. Une méthode de construction d'une sonde telle que définie dans l'une quelconque des revendications 1 et 3 à 12 qui comprend la combinaison des domaines A, B et C dans une liaison covalente.

18. Une méthode d'amplification d'un signal détectable utilisée pour détecter et quantifier un antigène dans un test immunologique qui comprend :
(a) la liaison directe ou indirecte à un analyte d'une sonde moléculaire bioohimique comprenant un domaine de liaison (A) qui est un polypeptide attaché de manière covalente à un second domaine (M) qui comprend une multiplicité de sous-unités oligonucléotidiques simple brin capables de s'hybrider avec un oligonucléotide marqué simple brin, ladite multiplicité de sous-unités oligonucléotidiques simple brin étant liées de manière covalente les unes aux autres ;
(b) l'élimination de la sondes non liée ;
(c) l'hybridation d'oligonucléotides marqués simple brin, qui comprennent une séquence nucléotidique qui est substantiellement complémentaire de la séquence de sous-unités de la sonde du domaine M, avec les sous-unités du second domaine ;
(d) l'élimination de l'oligonucléotides marqué non lié ; et
(e) la quantification de la quantité d'oligonucléotide marqué lié à la sonde.

19. Une méthode telle que revendiquée à la revendication 18 dans laquelle l'analyte est d'abord immobilisé, directement ou indirectement, sur un substrat solide.

20. Une méthode telle que revendiquée à la revendication 18 ou la revendication 19 dans laquelle le domaine A est un polypeptide capable de fonctionner comme un anticorps, et la sonde est liée directement ou indirectement à l'analyte via le domaine A.

21. Une méthode telle que revendiquée dans l'une quelconque des revendications 18 à 20 dans laquelle le polypeptide du domaine A se lie spécifiquement à un épitoge d'un anticorps qui se lie spécifiquement à l'analyte.

22. Une méthode telle que revendiquée dans l'une quelconque des revendications 18 à 21 utilisant une sonde dans laquelle chaque unité oligonucléotidique du second domaine, M, comprend environ 15 à 50 bases et a un contenu en GC dans la gamme d'environ 40% à 60%.

23. Une méthode telle que revendiquée dans l'une quelconque des revendications 18 à 22 dans laquelle le second domaine de ladite sonde a une structure linéaire.

24. Une méthode telle que revendiquée dans l'une quelconque des revendications 18 à 22 dans laquelle le second domaine de ladite sonde a une structure branchée.

25. Une méthode telle que revendiquée dans l'une quelconque des revendications 18 à 22 dans laquelle au moins une portion des sous-unités oligonucléotidiques du second domaine de ladite sonde est liée via une partie multifonctionnelle dérivée d'un composé de formule : 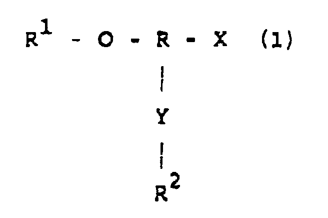 dans laquelle R est une partie organique, de préférence un acide nucléique, R¹ est un groupe protecteur d'hydroxyle qui peut être éliminé dans des conditions qui ne retirent pas l'acide nucléique de synthèse d'une phase solide et n'éliminent pas les groupes protecteurs d'azote exocyclique ou de phosphate, X est un groupe contenant du phosphore qui facilite la synthèse d'acide nucléique, Y est un radical dérivé d'un groupe nucléophile, et R² est R¹ ou un groupe bloquant ou protecteur qui peut être éliminé et remplacé par l'hydrogène sans affecter R¹.

26. Une méthode telle que revendiquée dans l'une quelconque des revendications 18 à 22 dans laquelle au moins une portion des sous-unités oligonucléotidiques du second domaine de ladite sonde sont liées via une partie multifonctionnelle dérivée d'un composé de formule : 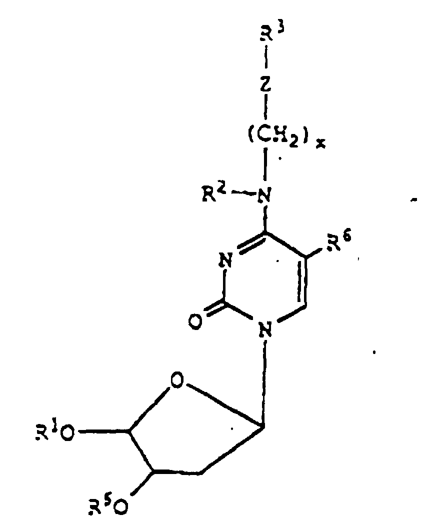 dans laquelle Z est un nucléophile, R¹ est un groupe protecteur qui est généralement résistant aux bases et sensible aux acides, R² est un hydrogène ou un méthyle, R³ est un groupe protecteur qui peut être éliminé et remplacé par l'hydrogène sans affecter R¹, R⁵ est un dérivé du phosphore qui permet l'addition de nucléotides en position 5' d'une chaîne oligonucléotidique durant la synthèse chimique, R⁶ est un méthyle, un hydrogène, I, Br, ou F, et X est un nombre entier dans la gamme de 1 à 8, inclus.

27. Une méthode telle que revendiquée dans l'une quelconque des revendications 18 à 22 dans laquelle au moins une portion des sous-unités oligonucléotidiques est liée via une partie multifonctionnelle dérivée d'un composé de formule : 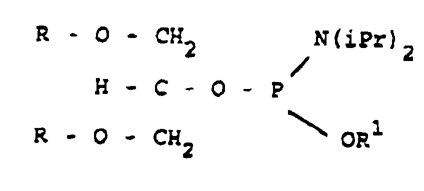 dans laquelle R est ledit groupe protecteur d'hydroxyle, iPr est un isopropyle, et R¹ est un méthyle ou un β-cyanoéthyle.

28. Une méthode de construction d'une sonde telle que définie dans l'une quelconque des revendications 18 et 20 à 27 qui comprend la liaison covalente des domaines A et M.

29. Une méthode d'amplification d'un signal détectable dans un essai immunologique de compétition qui comprend :
(a) la fourniture d'une sonde moléculaire comprenant :
(i) un premier domaine (A) qui est capable de fonctionner comme un immunogène ;
(il) un second domaine (B) qui est un polynucléotide double brin capable de fonctionner comme un promoteur pour une activité enzymatique d'ARN polymérase ADN dépendante ; et
(iii) un troisième domaine (C) qui est soit simple ou double brin et adjacent au second domaine, de sorte que le troisième domaine soit capable de fonctionner comme une matrice pour l'activité de promoteur du second domaine ;
(b) l'immobilisation de la sonde, directement ou indirectement, sur un substrat solide en présence d'un analyte entrant en compétition ;
(c) l'élimination de la sonde non liée ;
(d) la transcription de copies multiples d'oligomères d'ARN qui sont complémentaires de la séquence matrice, c', du troisième domaine, c, de la construction servant de sonde via une activité d'ARN polymérase ADN dépendante ; et
(e) la quantification des transcrits d'ARN.

30. Une méthode d'amplification d'un signal détectable dans un test immunologique de compétition qui comprend :
(a) la fourniture d'une sonde moléculaire comprenant :
(i) un premier domaine (A) qui est capable de fonctionner comme un immunogène et n'est pas un oligonucléotide ; et
(ii) un second domaine (M) comprenant une multiplicité de sous-unités oligonucléotidiques simple brin qui sont capables de se lier spécifiquement à une séquence d'acide nucléique simple brin d'intérêt.
(b) l'immobilisation de la sonde, directement ou indirectement, sur un substrat solide en présence d'un analyte entrant en compétition ;
(c) l'élimination de la sonde non liée ;
(d) l'hybridation d'oligonucléotides marqués simple brin, qui comprennent une séquence nucléotidique qui est substantiellement complémentaire de la séquence de la sous-unité de la sonde du domaine M, avec les sous-unités du second domaine ;
(e) l'élimination de 1'oligonucléotide marqué non lié ; et
(f) la quantification de la quantité d'oligonucléotide marqué lié à la sonde.

31. Une sonde moléculaire pour utilisation comme amplificateur de signal dans des tests immunologiques comprenant :
(a) un premier domaine (A) qui est un polypeptide et fonctionne comme un anticorps spécifique pour un antigène connu ;
(b) un second domaine (B) qui est un polynucléotide double brin capable de fonctionner comme un promoteur pour une activité enzymatique d'ARN polymérase ADN dépendante ; et
(c) un troisième domaine (C) qui est soit simple soit double brin et adjacent au second domaine, de façon que le troisième domaine soit capable de fonctionner comme une matrice pour l'activité du promoteur du second domaine.

32. Une sonde moléculaire pour utilisation comme amplificateur de signal dans des tests immunologiques comprenant :
(a) un premier domaine (A) comprenant un polypeptide capable de fonctionner comme un anticorps qui se lie spécifiquement à un analyte connu ; et
(b) un second domaine (M) comprenant une multiplicité de sous-unités oligonucléotidiques simple brin qui sont capables de se lier spécifiquement à une séquence d'acide nucléique simple brin d'intérêt, ladite multiplicité de sous-unités oligonucléotidiques simple brin étant liées de manière covalente les unes aux autres.
